# EUROPÄISCHE PATENTSCHRIFT

(11) **EP 3 059 223 B1**
(45) Veröffentlichungstag und Bekanntmachung des Hinweises auf die Patenterteilung: **28.06.2023**
(21) Anmeldenummer: 15155564.6
(22) Anmeldetag: 18.02.2015
(51) Int. Cl.: C07C 67/08, C07C 69/40, C07C 69/82, C08K 5/12

(54) **Herstellung von Estergemischen**
Preparation of ester mixtures
Préparation des mélanges d'esters

(43) Veröffentlichungstag der Anmeldung: 24.08.2016
(73) Patentinhaber: Evonik Operations GmbH, 45128 Essen (DE)
(72) Erfinder: Boeck, Florian Sebastian, 48143 Münster (DE); Gehlen, Carsten, 45772 Marl (DE); Grass, Michael, 45721 Haltern am See (DE); Woldt, Benjamin, 44892 Bochum (DE)
(74) Vertreter: Evonik Patent Association

(56) Entgegenhaltungen:
- WO-A1-2008/140177
- US-A1- 2014 096 703

## Beschreibung

Die vorliegende Erfindung betrifft ein Verfahren zur Herstellung eines Estergemisches gemäß den vorliegenden Ansprüchen, und die Verwendung des Verfahrens zur Einstellung
verarbeitungs- und/oder anwendungsrelevanter Eigenschaften eines Estergemisches gemäß Anspruch 9.

Die Verwendung von Estern der Terephthalsäure, der Phthalsäure, der Cyclohexandicarbonsäuren, der Adipinsäure, der Bernsteinsäure, der Citronensäure und anderer organischer Säuren als Weichmacher für Polymere ist seit längerem bekannt. Diese Ester werden hauptsächlich durch Veresterung der Säuren oder deren Derivate mit Alkoholen hergestellt.

Je nach der eingesetzten Säure beziehungsweise des eingesetzten Säurederivates und dem gewählten Alkohol variieren die Eigenschaften der resultierenden (ungemischten) Ester deutlich. Es resultieren Ester, welche als Weichmacher für verschiedenste Anwendungen geeignet sind, oftmals neben einer oder zwei sehr guten Eigenschaften jedoch auch ungünstige Eigenschaften aufweisen, welche dann den Einsatz des jeweiligen Esters stark beschränken können. Beispielsweise könnten Dioctylphthalat, Diisodecylphthalat, Diisononylphthalat, Trioctyltrimellitat und Triisononyltrimellitat gut in den unterschiedlichen Hochtemperaturanwendungen eingesetzt werden, da sie eine hohe Wärmebeständigkeit und geringe Flüchtigkeit aufweisen. Der Einsatz im Bereich dieser Anwendungen unterliege gemäß dem Dokument US 2014/0096703 A1 jedoch Beschränkungen, da diese Ester eine geringe Verträglichkeit mit den für diese Anwendungen eingesetzten Polymeren aufwiesen. Die Dibutyl-Derivate dieser Ester wiesen eine hohe Verträglichkeit mit Polymeren, beispielsweise PVC auf, kämen jedoch für Hochtemperaturanwendungen aufgrund ihrer geringen Wärmebeständigkeit nicht in Betracht. Um dennoch Weichmachersysteme bereitstellen zu können, welche eine gute Wärmebeständigkeit bei gleichzeitig guter Verträglichkeit aufweisen, schlägt das Dokument US 2014/0096703 A1 vor, ein Estergemisch aus C₈- und C₁₀-Estern der Terephthalsäure einzusetzen, welches neben den ungemischten Estern auch den Mischester, welcher einen C₈- und einen C₁₀-Alkoholrest aufweist, enthält.

Das Dokument KR 2013/0035493 A legt das Problem dar, dass Dibutylterephthalat zwar eine hohe Geschwindigkeit bei dem Eindringen in das Harz und beim Schmelzen aufwiese, jedoch einen unerwünscht hohen Migrationsverlust zeige, während bei Diethylhexylterephthalat kaum ein Migrationsverlust auftrete, das Eindringen in das Harz und das Schmelzen aber unakzeptabel lange dauere. Zur Verbesserung schlägt auch dieses Dokument vor, Estergemische umfassend den Mischester, welche einen C₄- und einen C₈-Alkoholrest enthält, einzusetzen.

Das Dokument WO 2008/140177 A1 schlägt vor, Estergemische aus C₈- und C₉-Estern der Terephthalsäure herzustellen, welche wiederum auch den Mischester enthalten, und beschreibt, dass eine Verbesserung der Verarbeitbarkeit der Weichmacher-Zubereitungen durch die Variation der Verhältnisse der einzelnen Ester im Estergemisch erreicht werden könne.

Bekannt sind auch Estergemische umfassend Mischester hergestellt aus Cyclohexandicarbonsäuren mit unterschiedlichen Alkoholen (WO 2011/115757 A1).

Auch Gemische von Zitronensäureestern, welche neben den entsprechenden ungemischten Estern auch Mischester mit C₅- und C₉-Alkoholresten enthalten, sind bekannt (US 8,431,638 B2).

Diese Estermischungen werden im Stand der Technik - unabhängig von ihrem Grundgerüst - durch das Verestern der entsprechenden Säuren oder Säurederivate mit einer Alkoholmischung bereitgestellt, welche die Alkoholreste der ungemischten Ester und des Mischesters beziehungsweise der Mischester liefert.

Wie beispielsweise in dem Dokument WO 2008/140177 A1 gezeigt wird, ist es jedoch nicht möglich, mittels der Zusammensetzung des Alkoholgemisches die Verteilung der resultierenden Ester gezielt einzustellen. So enthalten die in den Beispielen dieses Dokumentes hergestellten Estergemische die C₈- und C₉-Alkoholreste nicht in jenem Mengenverhältnis, in welchem diese Reste im eingesetzten Alkoholgemisch enthalten sind und wohl auch in das System eingeführt werden sollten. Auch bilden sich die C₈/C₈-Ester, die C_{8/}C₉-Ester und die C₉/C₉-Ester nicht in dem aufgrund von statistischen Überlegungen erwarteten Molverhältnissen, sondern in deutlich davon abweichenden Verhältnissen. Im Beispiel 1 des Dokument WO 2008/140177 A1 wird bei einem statistischen Erwartungswert von 25:50:25 (Annahme: äquimolarer Einbau der Alkoholreste) ein Molverhältnis der Ester von 10:54:36 erhalten, was einer nicht steuerbaren Abweichung um (|25-10| + |50-54| + |25-36| =) 30 Punkten entspricht (die Berechnung der Abweichung in Punkten wird später im Text erläutert). Während in diesem Beispiel die beiden Alkohole zwar in einem Molverhältnis von 1:1 eingesetzt werden, enthält das resultierende Estergemisch die beiden Alkoholreste in einem Molverhältnis von 37:63. Das Dokument schreibt diese Abweichungen vom statistischen Erwartungswert den unterschiedlichen Reaktionsgeschwindigkeiten der einzelnen Veresterungsreaktionen zu.

Dieses Problem der "Nichtsteuerbarkeit" der Estergemisch-Zusammensetzungen bei der Herstellung durch Veresterung der Terephthalsäure wird im Dokument KR 2013/0035493 A gelöst, indem die Ester des Gemisches getrennt hergestellt und im gewünschten Verhältnis abgemischt werden. Das Dokument enthält jedoch keine Offenbarung dazu, wie ein Mischester separat hergestellt werden kann, ohne dass sich gleichzeitig die beiden ungemischten Diester bilden. Dies scheint mit kommerziell vertretbarem Aufwand auch gar nicht möglich. Die Bereitstellung des puren Mischesters müsste durch dessen Abtrennung von den ungemischten Estern während eines Trennungsschrittes erfolgen. Soll dann dieser aufgereinigte Mischester mit den beiden ungemischten Estern gemäß Offenbarung des Dokumentes KR 2013/0035493 A abgemischt werden, müssen für die Herstellung eines Estergemisches beispielsweise zumindest drei Veresterungsreaktionen und eine anschließende Abmischung durchgeführt werden, was apparativ und zeitlich aufwändig ist.

Einen anderen Weg beschreibt das Dokument US 8,431,638 B2 für die Herstellung von gemischten Zitronensäureestern. Gemische umfassend diese Ester können hergestellt werden, indem Zitronensäurepentylester mit Zitronensäurenonylestern umgeestert werden. Diese Vorgehensweise erfordert dennoch drei getrennte Verfahrensschritte: zwei Veresterungen und eine Umesterung.

Der vorliegenden Erfindung lag nun die Aufgabe zugrunde, einige, vorzugsweise alle der oben genannten Nachteile des Standes der Technik zu überwinden. Mit Vorzug sollte ein Verfahren entwickelt werden, in welchem Gemische umfassend Mischester und ungemischte Ester mit vorbestimmter Mengenverteilung der Ester gezielt hergestellt werden können, wobei der apparative und zeitliche Aufwand so gering wie möglich gehalten werden soll.

Diese Aufgabe wird gelöst durch ein Verfahren zur Herstellung eines Estergemisches gemäß Anspruch 1.

Gegenstand der vorliegenden Erfindung ist deshalb ein Verfahren zur Herstellung von Estergemischen umfassend A(COOR¹)(COOR¹)ₓ, A(COOR¹)(COOR²)ₓ, A(COOR²)(COOR¹)ₓ und A(COOR²)(COOR²)ₓ durch Umsetzung des Esters A(COOR)ₓ₊₁, mit der Einschränkung, dass der Alkohol ROH des Restes R einen niedrigeren Siedepunkt bei einem bestimmten Druck aufweist als der Alkohol R¹OH des Restes R¹ bei demselben Druck, mit einer Menge (m₁ + s₁) R¹OH und einer Menge m₂ R²OH, wobei das Reaktionsgemisch zum Sieden erhitzt wird und
- A: für einen aromatischen, alicyclischen oder aliphatischen Rest steht,
- x: 1 ist,
- R¹ und R²: unabhängig voneinander für substituierte oder nichtsubstituierte Arylreste oder lineare oder verzweigte, substituierte oder nicht substituierte Alkylreste mit 3 bis 20 Kohlenstoffatomen stehen, wobei der Alkohol R¹OH einen niedrigeren Siedepunkt bei einem bestimmten Druck aufweist als der Alkohol R²OH bei demselben Druck,
- m₁ und m₂: den Mol-Äquivalenten der in A(COOH)ₓ₊₁ oder deren Derivaten einzuführenden Alkoholresten OR¹ und OR² entspricht und
- s₁: kleiner als m₁ + m₂ und gleichzeitig größer als 0,05 · (m₁ + m₂) ist.

Für x = 1 sind die Ester A(COOR¹)(COOR²)ₓ und A(COOR²)(COOR¹)ₓ ein und dieselbe Verbindung.

In der beanspruchten Ausführungsform wird in dem Verfahren der Ester A(COOR)ₓ₊₁, zu den Estern A(COOR¹)(COOR¹)ₓ, A(COOR¹)(COOR²)ₓ, A(COOR²)(COOR¹)ₓ und A(COOR²)(COOR²)ₓ umgeestert. Hierbei weist der Alkohol ROH des Restes R einen niedrigeren Siedepunkt bei einem bestimmten Druck auf als der Alkohol R¹OH des Restes R¹ bei demselben Druck.

Mit Vorzug werden die im Zuge des erfindungsgemäßen Verfahrens in A(COOR)ₓ₊₁ eingeführten Alkoholreste zu mindestens 95 Mol-%, bevorzugt zu mindestens 98 Mol-%, besonders bevorzugt zu mindestens 99 Mol-% und insbesondere zu 100 Mol-% Teil einer Esterfunktion.

In dem erfindungsgemäßen Verfahren wird A(COOR)ₓ₊₁ mit einem Gemisch der Alkohole R¹OH und R²OH umgesetzt. Überraschenderweise wurde festgestellt, dass dann die Zusammensetzung des resultierenden Estergemisches umfassend die ungemischten Ester A(COOR¹)(COOR¹)ₓ und A(COOR²)(COOR²)ₓ sowie die Mischester A(COOR¹)(COOR²)ₓ und A(COOR²)(COOR¹)ₓ gezielt - im Rahmen der sich für den Fall des vollständigen Einbaus der Alkoholreste OR² ergebenden Statistik - eingestellt werden kann, wenn der niedrigersiedende Alkohol R¹OH im Überschuss zu den in A(COOH)ₓ₊₁ oder deren Derivaten einzuführenden Alkoholresten OR¹ eingesetzt wird, die Menge des eingesetzten höhersiedenden Alkohols R²OH jedoch der Menge der einzuführenden Alkoholreste OR² entspricht. Eine Steuerung der Estergemisch-Zusammensetzung ist hingegen nicht möglich, wenn in einer Umesterung ein Überschuss des Alkoholgemisches (R¹OH + R²OH) in Bezug auf die einzuführenden Alkoholreste eingesetzt wird oder A(COOH)ₓ₊₁ oder ein Derivat davon mit einem Gemisch der Alkohole R¹OH und R²OH umgesetzt wird, wobei der höhersiedende Alkohol R²OH zu den in A(COOH)ₓ₊₁ oder deren Derivaten einzuführenden Alkoholresten OR² im Überschuss eingesetzt wird.

So ist es mittels des erfindungsgemäßen Verfahrens möglich, Estergemische bereitzustellen, in denen die unterschiedlichen Alkoholreste in einer vorbestimmten Mengenverteilung enthalten sind und in denen zudem die Mengenverteilung der enthaltenen Ester gezielt - im Rahmen der oben genannten Statistik - gesteuert werden kann. Somit ist es möglich, Estergemische bereitzustellen, deren Zusammensetzung geringere Abweichungen von der sich aufgrund der Statistik ergebenden Verteilung der Ester aufweist, als Estergemische, welche nach im Stand der Technik beschriebenen, ungesteuerten Verfahren hergestellt werden.

Für x = 1 ergeben sich die in der Tabelle 1 dargestellten statistischen Erwartungswerte der Estergemisch-Zusammensetzung.

**Tabelle 1: statistische Erwartungswerte Estergemisch-Zusammensetzung für x = 1**

| Einsatz Verhältnis R¹OH zu R²OH (m₁ : m₂) | | Erwartungsmenge A(COOR¹)₂ | Erwartungsmenge A(COOR¹)(COOR²) + A(COOR²)(COOR¹) | Erwartungsmenge A(COOR²)₂ |
|---|---|---|---|---|
| m₁ | m₂ | [Mol-%] | [Mol-%] | [Mol-%] |
| 9 | 1 | 81 | 18 | 1 |
| 8 | 2 | 64 | 32 | 4 |
| 7 | 3 | 49 | 42 | 9 |
| 6 | 4 | 36 | 48 | 16 |
| 5 | 5 | 25 | 50 | 25 |
| 4 | 6 | 16 | 48 | 36 |
| 3 | 7 | 9 | 42 | 49 |
| 2 | 8 | 4 | 32 | 64 |
| 1 | 9 | 1 | 18 | 81 |

Eine Quantifizierung der Abweichung der Estergemischzusammensetzung von der aus statistischen Überlegungen resultierenden Mengenverteilung der Ester im Estergemisch ist durch Summenbildung aller Beträge der Differenzen zwischen dem statistischem Erwartungswert, welcher sich ergibt, wenn man von einem vollständigen Einbau der Alkoholreste OR² ausgeht, und dem tatsächlichen Mol-Anteil jedes einzelnen Esters im Estergemisch für den Fall, dass sich die Summe der Mol-Anteile der oben genannten Ester im Estergemisch zu 100 addiert, möglich.

Mittels des erfindungsgemäßen Verfahrens gelingt es beispielsweise aus 5 Mol Dimethylterephthalat, 3,5 Mol Isopentanol und 9 Mol Isononanol gezielt ein Estergemisch bereitzustellen, in denen die Molverteilung der enthaltenen Ester lediglich um 1 Punkt vom statistischen Erwartungswert abweicht und der Anteil der Pentylreste im Estergemisch bis auf 0,3 % dem anvisierten Pentylanteil entspricht.

In dem erfindungsgemäßen Verfahren kann der Rest A für aromatische, alicyclische oder aliphatische Reste stehen. In einer Ausführungsform steht A für einen aliphatischen Rest mit 4 bis 12 Kohlenstoffatomen, welcher 2 oder 3 Säurefunktionen umfasst und optional weitere funktionelle Gruppen trägt. In einer anderen Ausführungsform steht A für einen ungesättigten Alkylrest, welcher 4 bis 12 Kohlenstoffatome enthält, wobei er 2 oder 3 Säurefunktionen aufweist und optional weitere funktionelle Gruppen trägt. In einer weiteren Ausführungsform steht A für einen alicyclischen Rest, welcher 4 bis 8 Kohlenwasserstoffatome aufweist, an welche 2 oder 3 Carbonsäurefunktionen und optional weitere funktionale Reste angebunden sind. In einer weiteren Ausführungsform steht A für einen aromatischen Rest mit 2 oder 3 Carbonsäurefunktionen und optional weiteren funktionellen Gruppen.

Bevorzugt handelt es sich bei dem mittels des erfindungsgemäßen Verfahren hergestellten Estergemisch um ein Estergemisch der Phthalsäure, der Terephthalsäure, der Isophthalsäure, der 1,2-, 1,3- oder 1,4- Cyclohexandicarbonsäure, der Adipinsäure, der Sebacinsäure, der Maleinsäure, der Bernsteinsäure, der Furandicarbonsäure oder der Citronensäure.

Die Reste R¹ und R² können unabhängig voneinander ausgewählt werden aus substituierten oder nichtsubstituierten Arylresten, beispielsweise Benzylresten und linearen oder verzweigten, substituierten oder nicht substituierten Alkylresten mit 3 bis 20 Kohlenstoffatomen, solange der Alkohol R¹OH einen niedrigeren Siedepunkt bei einem bestimmten Druck aufweist als der Alkohol R²OH bei demselben Druck. In einer Ausführungsform sind die Reste R¹ und R² ausgewählt aus linearen oder verzweigten, substituierten oder nicht substituierten Alkylresten mit 3 bis 20 Kohlenstoffatomen, wobei vorzugsweise einer der Reste R¹ oder R² oder beide Reste R¹ und R² weitere funktionelle Gruppen, beispielsweise eine oder mehrere Mehrfachbindungen, Ether-, Aldehyd-, Keton-, Hydroxy- und/oder Halogenidgruppen enthält beziehungsweise enthalten. In einer anderen Ausführungsform sind die Reste R¹ und R² unabhängig voneinander ausgewählt aus Alkylresten, welche 3 bis 20, vorzugsweise 4 bis 15 und insbesondere 5 bis 11 Kohlenstoffatome enthalten, wobei die Alkylreste bevorzugt keine weiteren funktionellen Gruppen einschließlich Mehrfachbindungen aufweisen. Vorzugsweise sind R¹ und R² unabhängig voneinander ausgewählt aus Propyl-, Butyl-, tert-Butyl-, Isobutyl-, 2-Methylbutyl-, 3-Methylbutyl-, n-Pentyl-, Isopentyl-, Hexyl-, Heptyl-, Isoheptyl-, Octyl-, Isooctyl-, 2-Ethylhexyl-, Nonyl-, n-Nonyl-, Isononyl-, Decyl-, Isodecyl-, 2-Propylheptyl-, Undecyl- und Tridecyl- Resten.

Mit Vorzug ist hierbei der Rest R¹ ausgewählt aus Propyl-, Butyl-, tert-Butyl-, Isobutyl-, 2-Methylbutyl-, 3-Methylbutyl-, n-Pentyl-, Isopentyl-, Hexyl-, Heptyl- und Isoheptyl-Resten und gleichzeitig ist der Rest R² ausgewählt aus n-Pentyl-, Isopentyl-, Hexyl-, Heptyl-, Isoheptyl-, Octyl-, Isooctyl-, 2-Ethylhexyl-, Nonyl-, n-Nonyl-, Isononyl-, Decyl-, Isodecyl-, 2-Propylheptyl-, Undecyl- und Tridecyl- Resten, jedoch mit der Maßgabe, dass der Alkohol R¹OH des Restes R¹ einen niedrigeren Siedepunkt bei einem bestimmten Druck aufweist als der Alkohol R²OH des Restes R² bei demselben Druck.

Es hat sich herausgestellt, dass das Verfahren mit besonders geringen Abweichungen von dem aufgrund der stöchiometrischen Einsatzmengen m₁ und m₂ statistisch vorgestimmten Molverhältnis der gebildeten ungemischten Ester und Mischester durchgeführt werden kann, wenn die Siedepunkte der Alkohole R¹OH und R²OH sich um mindestens 10 °C, vorzugsweise um mindestens 25 °C und insbesondere um mindestens 40 °C unterscheiden. Bevorzugt weist der Alkohol R¹OH bei 1013 hPa einen um mindestens 10 °C, besonders bevorzugt einen um mindestens 20 °C, bevorzugt dazu einen um mindestens 30 °C, mit Vorzug einen um mindestens 40 °C, mit besonderem Vorzug einen um mindestens 50 °C und insbesondere einen um mindestens 60 °C niedrigeren Siedepunkt auf als der Alkohol R²OH bei demselben Druck.

Handelt es sich bei dem Alkohol R¹OH und/oder bei dem Alkohol R²OH um ein Isomerengemisch, so ist dies im Rahmen dieses Textes - wie in der Technischen Chemie üblich - durch das Präfix "/*so*" gekennzeichnet. Isomerengemische weisen keinen scharfen Siedepunkt auf, sondern besitzen einen Siedebereich. Im Fall von Isomerengemischen wird die Differenz der Siedepunkte im Rahmen der vorliegenden Erfindung durch Differenzbildung zwischen der unteren Siedebereichsgrenze des höhersiedenden Alkohols und der oberen Siedebereichsgrenze des niedrigersiedenden Alkohols bestimmt.

Mit Vorzug werden die Alkohole R¹OH und R²OH in einem solchen Verhältnis zueinander in dem erfindungsgemäßen Verfahren eingesetzt, dass das Verhältnis von m₁ zu m₂ (m₁:m₂) im Bereich von 1:9 bis 9:1, vorzugsweise im Bereich von 2:8 bis 8:2, besonders bevorzugt im Bereich von 3:7 bis 7:3 und insbesondere im Bereich von 4:6 bis 6:4 liegt. Werden die Alkohole R¹OH und R²OH in einem Verhältnis m₁:m₂ größer 1,5:8,5, vorzugsweise größer 2:8 und insbesondere größer 2,5:7,5 eingesetzt, können Estergemische erhalten werden, welche gegenüber dem A(COOR²)(COOR²)ₓ deutlich günstigere, das heißt niedrigere, Geliertemperaturen aufweisen und sich gleichzeitig gegenüber A(COOR¹)(COOR¹)ₓ durch eine geringere und damit verbesserte Flüchtigkeit auszeichnen. Estergemische, welche besonders gut mit Polymeren, beispielsweise PVC verarbeitet werden können, können erhalten werden, wenn die Alkohole R¹OH und R²OH im einem Verhältnis m₁:m₂ eingesetzt werden, welches größer ist als 1,5:8,5, bevorzugt größer ist als 2:8 und insbesondere größer ist als 2,5:7,5. Estergemische, welche sich zur Herstellung von Estergemisch- und Polymer-haltigen Mitteln mit guter Lagerfähigkeit eignen, können erhalten werden, wenn die Alkohole R¹OH und R²OH in einem Verhältnis m₁:m₂ kleiner 8:2, vorzugsweise kleiner 7,5:2,5 und insbesondere kleiner 7:3 eingesetzt werden. Eine weitere Verbesserung der Eigenschaften im Bereich der Flüchtigkeit wird möglich, wenn die Alkohole R¹OH und R²OH in einem Verhältnis m₁:m₂ kleiner 4:6, vorzugsweise kleiner 3,5:6,5, weiter bevorzugt kleiner 3:7, ganz besonders bevorzugt kleiner 2,5:7,5, dazu bevorzugt kleiner 2:8 und insbesondere kleiner 1,5:8,5 eingesetzt werden. Bei der Bildung all dieser Verhältnisse ist die "Überschussmenge" s₁ an R¹OH nicht berücksichtigt.

Die Menge s₁ an R¹OH, welche über die Menge m₁ der in A(COOR)ₓ₊₁ einzuführenden Alkoholfunktionen OR¹ hinausgeht, kann in dem erfindungsgemäßen Verfahren als Lösungsmittel wirken. Wird in dem erfindungsgemäßen Verfahren Wasser gebildet, so wirkt die Menge s₁ an R¹OH vorzugsweise als Schleppmittel für das Wasser, welches mit R¹OH als azeotropes Gemisch abdestilliert werden kann. Die Menge s₁ ist kleiner als m₁ + m₂, mit Vorzug kleiner als 0,6 · (m₁ + m₂), bevorzugt keiner als 0,5 · (m₁ + m₂), besonders bevorzugt kleiner als 0,4 · (m₁ + m₂), weiter bevorzugt kleiner als 0,3 · (m₁ + m₂) und insbesondere kleiner als 0,25 · (m₁ + m₂). Die Menge s₁ ist gleichzeitig größer als 0,05 · (m₁ + m₂), bevorzugt größer als 0,10 · (m₁ + m₂), besonders bevorzugt größer als 0,15 · (m₁ + m₂) und insbesondere größer als 0,20 · (m₁ + m₂).. Die Menge s₁ ist also kleiner als (m₁ + m₂), mit Vorzug kleiner als 0,6 · (m₁ + m₂), besonders bevorzugt keiner als 0,5 · (m₁ + m₂) und insbesondere kleiner als 0,4 · (m₁ + m₂) und gleichzeitig größer als 0,05 · (m₁ + m₂), bevorzugt größer als 0,10. (m₁ + m₂) und insbesondere größer als 0,15 · (m₁ + m₂).

Bevorzugt wird als Terephthalsäurederivat in dem erfindungsgemäßen Verfahren Dimethylterephthalat eingesetzt.

Ein bevorzugter Gegenstand der vorliegenden Erfindung ist ein Verfahren zur Herstellung von Estergemischen umfassend Dipentylterephthalat, Diisononylterephthalat und Pentyl(isononyl)terephthalat durch Umsetzung von Terephthalsäure oder Derivaten davon, welche keine Estergruppen COOR mit einem Rest R enthalten, dessen Alkohol ROH einen höheren Siedepunkt bei einem bestimmten Druck aufweist als Pentanol bei demselben Druck, mit einer Menge (m₁ + s₁) Pentanol und einer Menge m₂ Isononanol, wobei das Reaktionsgemisch zum Sieden erhitzt wird und
- m₁ und m₂: den Mol-Äquivalenten der in Terephthalsäure oder ihren Derivaten einzuführenden Alkoholresten von Pentanol und Isononanol entspricht und
- s₁: größer als 0 ist und insbesondere im Bereich des 0,05- bis 0,60-fachen von (m₁ + m₂) liegt.

Ein bevorzugter Gegenstand der vorliegenden Erfindung ist ein Verfahren zur Herstellung von Estergemischen umfassend Dipentylterephthalat, Dipropylheptylterephthalat und Pentyl(propylheptyl)terephthalat durch Umsetzung von Terephthalsäure oder Derivaten davon, welche keine Estergruppen COOR mit einem Rest R enthalten, dessen Alkohol ROH einen höheren Siedepunkt bei einem bestimmten Druck aufweist als Pentanol bei demselben Druck, mit einer Menge (m₁ + s₁) Pentanol und einer Menge m₂ Propylheptanol, wobei das Reaktionsgemisch zum Sieden erhitzt wird und
- m₁ und m₂: den Mol-Äquivalenten der in Terephthalsäure oder ihren Derivaten einzuführenden Alkoholresten von Pentanol und Propylheptanol entspricht und
- s₁: größer als 0 ist und insbesondere im Bereich des 0,05- bis 0,60-fachen von (m₁ + m₂) liegt.

Ein bevorzugter Gegenstand der vorliegenden Erfindung ist ein Verfahren zur Herstellung eines Estergemisches der 1,2-, 1,3- oder 1,4-Ester der Cyclohexandicarbonsäure umfassend Cyclohexandicarbonsäuredipentylester, Cyclohexandicarbonsäurediisononylester und Cyclohexandicarbonsäurepentyl(isononyl)ester durch Umsetzung von 1,2-, 1,3- oder 1,4-Cyclohexandicarbonsäure oder Derivaten davon, welche keine Estergruppen COOR mit einem Rest R enthalten, dessen Alkohol ROH einen höheren Siedepunkt bei einem bestimmten Druck aufweist als Pentanol bei demselben Druck, mit einer Menge (m₁ + s₁) Pentanol und einer Menge m₂ Isononanol, wobei das Reaktionsgemisch zum Sieden erhitzt wird und
- m₁ und m₂: den Mol-Äquivalenten der in 1,2-, 1,3- oder 1,4-Cyclohexandicarbonsäure oder ihren Derivaten einzuführenden Alkoholresten von Pentanol und Isononanol entspricht und
- s₁: größer als 0 ist und insbesondere im Bereich des 0,05- bis 0,60-fachen von (m₁ + m₂) liegt.

Ein bevorzugter Gegenstand der vorliegenden Erfindung ist ein Verfahren zur Herstellung eines Estergemisches der 1,2-, 1,3- oder 1,4-Ester der Cyclohexandicarbonsäure umfassend Cyclohexandicarbonsäuredipentylester, Cyclohexandicarbonsäuredipropylheptylester und Cyclohexandicarbonsäurepentyl(propylheptyl)ester durch Umsetzung von 1,2-, 1,3- oder 1,4-Cyclohexandicarbonsäure oder Derivaten davon, welche keine Estergruppen COOR mit einem Rest R enthalten, dessen Alkohol ROH einen höheren Siedepunkt bei einem bestimmten Druck aufweist als Pentanol bei demselben Druck, mit einer Menge (m₁ + s₁) Pentanol und einer Menge m₂ Propylheptanol, wobei das Reaktionsgemisch zum Sieden erhitzt wird und
- m₁ und m₂: den Mol-Äquivalenten der in 1,2-, 1,3- oder 1,4-Cyclohexandicarbonsäure oder ihren Derivaten einzuführenden Alkoholresten von Pentanol und Propylheptanol entspricht und
- s₁: größer als 0 ist und insbesondere im Bereich des 0,05- bis 0,60-fachen von (m₁ + m₂) liegt.

Ein bevorzugter Gegenstand der vorliegenden Erfindung ist ein Verfahren zur Herstellung von Estergemischen umfassend Dipentylsuccinat, Diisononylsuccinat und Pentyl(isononyl)succinat durch Umsetzung von Bernsteinsäure oder Derivaten davon, welche keine Estergruppen COOR mit einem Rest R enthalten, dessen Alkohol ROH einen höheren Siedepunkt bei einem bestimmten Druck aufweist als Pentanol bei demselben Druck, mit einer Menge (m₁ + s₁) Pentanol und einer Menge m₂ Isononanol, wobei das Reaktionsgemisch zum Sieden erhitzt wird und
- m₁ und m₂: den Mol-Äquivalenten der in Bernsteinsäure oder ihren Derivaten einzuführenden Alkoholresten von Pentanol und Isononanol entspricht und
- s₁: größer als 0 ist und insbesondere im Bereich des 0,05- bis 0,60-fachen von (m₁ + m₂) liegt.

Ein bevorzugter Gegenstand der vorliegenden Erfindung ist ein Verfahren zur Herstellung von Estergemischen umfassend Dipentylsuccinat, Dipropylheptylsuccinat und Pentyl(propylheptyl)succinat durch Umsetzung von Bernsteinsäure oder Derivaten davon, welche keine Estergruppen COOR mit einem Rest R enthalten, dessen Alkohol ROH einen höheren Siedepunkt bei einem bestimmten Druck aufweist als Pentanol bei demselben Druck, mit einer Menge (m₁ + s₁) Pentanol und einer Menge m₂ Propylheptanol, wobei das Reaktionsgemisch zum Sieden erhitzt wird und
- m₁ und m₂: den Mol-Äquivalenten der in Bernsteinsäure oder ihren Derivaten einzuführenden Alkoholresten von Pentanol und Propylheptanol entspricht und
- s₁: größer als 0 ist und insbesondere im Bereich des 0,05- bis 0,60-fachen von (m₁ + m₂) liegt.

Ein bevorzugter Gegenstand der vorliegenden Erfindung ist ein Verfahren zur Herstellung von Estergemischen umfassend Tripentylcitrat, Diisononyl(pentyl)citrat, Dipentyl(isononyl)citrat und Triisononylcitrat durch Umsetzung von Citronensäure oder Derivaten davon, welche keine Estergruppen COOR mit einem Rest R enthalten, dessen Alkohol ROH einen höheren Siedepunkt bei einem bestimmten Druck aufweist als Pentanol bei demselben Druck, mit einer Menge (m₁ + s₁) Pentanol und einer Menge m₂ Isononanol, wobei das Reaktionsgemisch zum Sieden erhitzt wird und
- m₁ und m₂: den Mol-Äquivalenten der in Citronensäure oder ihren Derivaten einzuführenden Alkoholresten von Pentanol und Isononanol entspricht und
- s₁: größer als 0 ist und insbesondere im Bereich des 0,05- bis 0,60-fachen von (m₁ + m₂) liegt.

Ein bevorzugter Gegenstand der vorliegenden Erfindung ist ein Verfahren zur Herstellung von Estergemischen umfassend Tripentylcitrat, Dipropylheptyl(pentyl)citrat, Dipentyl(propylheptyl)citrat und Tripropylheptylcitrat durch Umsetzung von Citronensäure oder Derivaten davon, welche keine Estergruppen COOR mit einem Rest R enthalten, dessen Alkohol ROH einen höheren Siedepunkt bei einem bestimmten Druck aufweist als Pentanol bei demselben Druck, mit einer Menge (m₁ + s₁) Pentanol und einer Menge m₂ Propylheptanol, wobei das Reaktionsgemisch zum Sieden erhitzt wird und
- m₁ und m₂: den Mol-Äquivalenten der in Citronensäure oder ihren Derivaten einzuführenden Alkoholresten von Pentanol und Propylheptanol entspricht und
- s₁: größer als 0 ist und insbesondere im Bereich des 0,05- bis 0,60-fachen von (m₁ + m₂) liegt.

Vorzugsweise weist das in dem erfindungsgemäßen Verfahren, insbesondere das in diesen Ausführungsformen eingesetzte Isononanol einen durchschnittlichen Verzweigungsgrad von 1,0 bis 2,2 auf. Der durchschnittliche Verzweigungsgrad wird dabei bestimmt wie im Dokument US 2010/305255 A1 beschrieben. In einer anderen Ausführungsform enthält das in dem erfindungsgemäßen Verfahren, insbesondere das in obigen Ausführungsformen eingesetzte Isononanol weniger als 25 Mol-%, insbesondere weniger als 15 Mol-% Alkohole mit einer Kohlenstoffatomanzahl, welche kleiner oder größer ist als 9. Besonders bevorzugt weist das in dem erfindungsgemäßen Verfahren, insbesondere das in diesen Ausführungsformen eingesetzte Isononanol einen durchschnittlichen Verzweigungsgrad von 1,0 bis 2,2 auf und enthält gleichzeitig weniger als 25 Mol-%, insbesondere weniger als 15 Mol-% Alkohole mit einer Kohlenstoffatomanzahl, welche kleiner oder größer ist als 9 ist.

Unabhängig davon werden in dem erfindungsgemäßen Verfahren, insbesondere in den oben beschriebenen Ausführungsformen vorzugsweise Ester eingesetzt, welche n-Pentyl-, 2-Methylbutyl- und/oder 3-Methylbutyl-Reste enthalten. Eine bevorzugte Ausführungsform liegt vor, wenn in den oben beschriebenen Ausführungsformen die Pentyl-Reste Isomerengemische und somit Isopentyl-Reste sind. Mit Vorzug liegt der Anteil der n-Pentyl-Reste basierend auf allen enthaltenen Pentyl-Resten bei mindestens 10 Mol-% oder 20 Mol-%, bevorzugt bei mindestens 30 Mol-%, besonders bevorzugt bei mindestens 40 Mol-%, ganz besonders bevorzugt bei mindestens 50 Mol-% und insbesondere bei mindestens 60 Mol-%, was mit dem Vorteil einer geringeren und damit für die Verarbeitung von Plastisolen günstigeren Viskosität verknüpft sein kann. In einer bevorzugten Ausführungsform liegt der der Anteil der n-Pentyl-Reste basierend auf allen enthaltenen Pentyl-Resten zwischen 10 und 90 Mol-%, bevorzugt bei 20 bis 80 Mol-% und insbesondere bei 30 bis 70 Mol-%.

Bei den Propylheptyl-Resten handelt es sich vorzugsweise um 2-Propylheptyl-Reste.

Ein bevorzugter Gegenstand der vorliegenden Erfindung ist ein Verfahren zur Herstellung von Estergemischen umfassend Dibutylterephthalat, Dioctylterephthalat und Butyl(octyl)terephthalat durch Umsetzung von Terephthalsäure oder Derivaten davon, welche keine Estergruppen COOR mit einem Rest R enthalten, dessen Alkohol ROH einen höheren Siedepunkt bei einem bestimmten Druck aufweist als Butanol bei demselben Druck, mit einer Menge (m₁ + s₁) Butanol und einer Menge m₂ Octanol, wobei das Reaktionsgemisch zum Sieden erhitzt wird und
- m₁ und m₂: den Mol-Äquivalenten der in Terephthalsäure oder ihren Derivaten einzuführenden Alkoholresten von Butanol und Octanol entspricht und
- s₁: größer als 0 ist und insbesondere im Bereich des 0,05- bis 0,60-fachen von (m₁ + m₂) liegt.

Bevorzugt handelt es sich hierbei bei den Octyl-Resten um Ethylhexyl-Reste, insbesondere um 2-Ethylhexyl-Reste und/oder bei den Butyl-Resten um n-Butyl-Reste.

Ein bevorzugter Gegenstand der vorliegenden Erfindung ist ein Verfahren zur Herstellung von Estergemischen umfassend Dinonylterephthalat, Didecylterephthalat und Nonyl(decyl)terephthalat durch Umsetzung von Terephthalsäure oder Derivaten davon, welche keine Estergruppen COOR mit einem Rest R enthalten, dessen Alkohol ROH einen höheren Siedepunkt bei einem bestimmten Druck aufweist als Nonanol bei demselben Druck, mit einer Menge (m₁ + s₁) Nonanol und einer Menge m₂ Decanol, wobei das Reaktionsgemisch zum Sieden erhitzt wird und
- m₁ und m₂: den Mol-Äquivalenten der in Terephthalsäure oder ihren Derivaten einzuführenden Alkoholresten von Nonanol und Decanol entspricht und
- s₁: größer als 0 ist und insbesondere im Bereich des 0,05- bis 0,60-fachen von (m₁ + m₂) liegt.

Bevorzugt handelt es sich hierbei bei den Nonyl-Resten um Isononyl-Reste und/oder bei den Decyl-Resten um Propylheptyl-Reste, insbesondere um 2-Propylheptyl-Reste.

Ein bevorzugter Gegenstand der vorliegenden Erfindung ist ein Verfahren zur Herstellung von Estergemischen umfassend Dioctylterephthalat, Didecylterephthalat und Octyl(decyl)terephthalat durch Umsetzung von Terephthalsäure oder Derivaten davon, welche keine Estergruppen COOR mit einem Rest R enthalten, dessen Alkohol ROH einen höheren Siedepunkt bei einem bestimmten Druck aufweist als Octanol bei demselben Druck, mit einer Menge (m₁ + s₁) Octanol und einer Menge m₂ Decanol, wobei das Reaktionsgemisch zum Sieden erhitzt wird und
- m₁ und m₂: den Mol-Äquivalenten der in Terephthalsäure oder ihren Derivaten einzuführenden Alkoholresten von Octanol und Decanol entspricht und
- s₁: größer als 0 ist und insbesondere im Bereich des 0,05- bis 0,60-fachen von (m₁ + m₂) liegt.

Bevorzugt handelt es sich hierbei bei den Octyl-Resten um Ethylhexyl-Reste, insbesondere um 2-Ethylhexyl-Reste und/oder bei den Decyl-Resten um Propylheptyl-Reste, insbesondere um 2-Propylheptyl-Reste.

Ein bevorzugter Gegenstand der vorliegenden Erfindung ist ein Verfahren zur Herstellung von Estergemischen umfassend Diheptylterephthalat, Dinonylterephthalat und Heptyl(nonyl)terephthalat durch Umsetzung von Terephthalsäure oder Derivaten davon, welche keine Estergruppen COOR mit einem Rest R enthalten, dessen Alkohol ROH einen höheren Siedepunkt bei einem bestimmten Druck aufweist als Heptanol bei demselben Druck, mit einer Menge (m₁ + s₁) Heptanol und einer Menge m₂ Nonanol, wobei das Reaktionsgemisch zum Sieden erhitzt wird und
- m₁ und m₂: den Mol-Äquivalenten der in Terephthalsäure oder ihren Derivaten einzuführenden Alkoholresten von Heptanol und Nonanol entspricht und
- s₁: größer als 0 ist und insbesondere im Bereich des 0,05- bis 0,60-fachen von (m₁ + m₂) liegt.

Bevorzugt handelt es sich hierbei bei den Nonyl-Resten um Isononyl-Reste und /oder bei den Heptyl-Resten um Isoheptyl-Reste.

Ein bevorzugter Gegenstand der vorliegenden Erfindung ist ein Verfahren zur Herstellung eines Estergemisches der 1,2-, 1,3- oder 1,4-Ester der Cyclohexandicarbonsäure umfassend Cyclohexandicarbonsäuredibutylester, Cyclohexandicarbonsäuredioctylester und Cyclohexandicarbonsäurebutyl(octyl)ester durch Umsetzung von 1,2-, 1,3- oder 1,4-Cyclohexandicarbonsäure oder Derivaten davon, welche keine Estergruppen COOR mit einem Rest R enthalten, dessen Alkohol ROH einen höheren Siedepunkt bei einem bestimmten Druck aufweist als Butanol bei demselben Druck, mit einer Menge (m₁ + s₁) Butanol und einer Menge m₂ Octanol, wobei das Reaktionsgemisch zum Sieden erhitzt wird und
- m₁ und m₂: den Mol-Äquivalenten der in 1,2-, 1,3- oder 1,4-Cyclohexandicarbonsäure oder ihren Derivaten einzuführenden Alkoholresten von Butanol und Octanol entspricht und
- s₁: größer als 0 ist und insbesondere im Bereich des 0,05- bis 0,60-fachen von (m₁ + m₂) liegt.

Bevorzugt handelt es sich hierbei bei den Octyl-Resten um Ethylhexyl-Reste, insbesondere um 2-Ethylhexyl-Reste und/oder bei den Butyl-Resten um n-Butyl-Reste.

Ein bevorzugter Gegenstand der vorliegenden Erfindung ist ein Verfahren zur Herstellung eines Estergemisches der 1,2-, 1,3- oder 1,4-Ester der Cyclohexandicarbonsäure umfassend Cyclohexandicarbonsäuredinonylester, Cyclohexandicarbonsäuredidecylester und Cyclohexandicarbonsäurenonyl(decyl)ester durch Umsetzung von 1,2-, 1,3- oder 1,4-Cyclohexandicarbonsäure oder Derivaten davon, welche keine Estergruppen COOR mit einem Rest R enthalten, dessen Alkohol ROH einen höheren Siedepunkt bei einem bestimmten Druck aufweist als Nonanol bei demselben Druck, mit einer Menge (m₁ + s₁) Nonanol und einer Menge m₂ Decanol, wobei das Reaktionsgemisch zum Sieden erhitzt wird und
- m₁ und m₂: den Mol-Äquivalenten der in 1,2-, 1,3- oder 1,4-Cyclohexandicarbonsäure oder ihren Derivaten einzuführenden Alkoholresten von Nonanol und Decanol entspricht und
- s₁: größer als 0 ist und insbesondere im Bereich des 0,05- bis 0,60-fachen von (m₁ + m₂) liegt.

Bevorzugt handelt es sich hierbei bei den Nonyl-Resten um Isononyl-Reste und/oder bei den Decyl-Resten um Propylheptyl-Reste, insbesondere um 2-Propylheptyl-Reste.

Ein bevorzugter Gegenstand der vorliegenden Erfindung ist ein Verfahren zur Herstellung eines Estergemisches der 1,2-, 1,3- oder 1,4-Ester der Cyclohexandicarbonsäure umfassend Cyclohexandicarbonsäuredioctylester, Cyclohexandicarbonsäuredidecylester und Cyclohexandicarbonsäureoctyl(decyl)ester durch Umsetzung von 1,2-, 1,3- oder 1,4-Cyclohexandicarbonsäure oder Derivaten davon, welche keine Estergruppen COOR mit einem Rest R enthalten, dessen Alkohol ROH einen höheren Siedepunkt bei einem bestimmten Druck aufweist als Octanol bei demselben Druck, mit einer Menge (m₁ + s₁) Octanol und einer Menge m₂ Decanol, wobei das Reaktionsgemisch zum Sieden erhitzt wird und
- m₁ und m₂: den Mol-Äquivalenten der in 1,2-, 1,3- oder 1,4-Cyclohexandicarbonsäure oder ihren Derivaten einzuführenden Alkoholresten von Octanol und Decanol entspricht und
- s₁: größer als 0 ist und insbesondere im Bereich des 0,05- bis 0,60-fachen von (m₁ + m₂) liegt.

Bevorzugt handelt es sich hierbei bei den Octyl-Resten um Ethylhexyl-Reste, insbesondere um 2-Ethylhexyl-Reste und/oder bei den Decyl-Resten um Propylheptyl-Reste, insbesondere um 2-Propylheptyl-Reste.

Ein bevorzugter Gegenstand der vorliegenden Erfindung ist ein Verfahren zur Herstellung eines Estergemisches der 1,2-, 1,3- oder 1,4-Ester der Cyclohexandicarbonsäure umfassend Cyclohexandicarbonsäurediheptylester, Cyclohexandicarbonsäuredinonylester und Cyclohexandicarbonsäureheptyl(nonyl)ester durch Umsetzung von 1,2-, 1,3- oder 1,4-Cyclohexandicarbonsäure oder Derivaten davon, welche keine Estergruppen COOR mit einem Rest R enthalten, dessen Alkohol ROH einen höheren Siedepunkt bei einem bestimmten Druck aufweist als Heptanol bei demselben Druck, mit einer Menge (m₁ + s₁) Heptanol und einer Menge m₂ Nonanol, wobei das Reaktionsgemisch zum Sieden erhitzt wird und
- m₁ und m₂: den Mol-Äquivalenten der in 1,2-, 1,3- oder 1,4-Cyclohexandicarbonsäure oder ihren Derivaten einzuführenden Alkoholresten von Heptanol und Nonanol entspricht und
- s₁: größer als 0 ist und insbesondere im Bereich des 0,05- bis 0,60-fachen von (m₁ + m₂) liegt.

Bevorzugt handelt es sich hierbei bei den Nonyl-Resten um Isononyl-Reste und/oder bei den Heptyl-Resten um Isoheptyl-Reste.

Besonders bevorzugt weist auch das in den voranstehenden Ausführungsformen des erfindungsgemäßen Verfahren eingesetzte Isononanol einen durchschnittlichen Verzweigungsgrad von 1,0 bis 2,2 auf und enthält vorzugsweise zudem gleichzeitig weniger als 25 Mol-%, insbesondere weniger als 15 Mol-% Alkohole mit einer Kohlenstoffatomanzahl, welche kleiner oder größer ist als 9 ist.

In dem erfinderischen Verfahren wird vorzugsweise keine Überschussmenge an R²OH eingesetzt, sondern nur die Menge an R²OH, welche der Menge an in A(COOR)ₓ₊₁ einzubauenden Alkoholresten OR², insbesondere der Menge an in A A(COOR)ₓ₊₁ als Teil von COOR²-Esterfunktionen einzubauenden Alkoholresten OR² entspricht. "Keine Überschussmenge" bedeutet hier, dass vorzugsweise weniger als 0,2 Mol-Äquivalente, bevorzugt weniger als 0,1 Mol-Äquivalente und insbesondere weniger als 0,05 Mol-Äquivalente basierend auf der Anzahl der eingesetzten Menge an R²OH nicht als OR² in A(COOR)ₓ₊₁ eingebaut wird. Dementsprechend werden vorzugsweise mindestens 0,8 Mol-Äquivalente, bevorzugt mindestens 0,9 Mol-Äquivalente, weiter bevorzugt mindestens 0,95 Mol-Äquivalente und insbesondere mindestens 0,98 Mol-Äquivalente des in dem erfindungsgemäßen Verfahren eingesetzten Alkohols R²OH als Alkoholrest OR² in A(COOR)ₓ₊₁ eingeführt.

Die gewünschte Zusammensetzung des herzustellenden Estergemisches kann besonders gut gesteuert und das Verfahrensprodukt nach einem geringeren Aufarbeitungsaufwand direkt als Weichmacher oder Weichmacherkomponente genutzt werden, wenn die in dem erfindungsgemäßen Verfahren eingesetzten Komponenten umfassend A(COOR)ₓ₊₁ und das Alkoholgemisch von R¹OH und R¹OH weniger als 50 Vol.-%, vorzugsweise weniger als 35 Vol.-% und insbesondere weniger als 20 Vol.-%, weiter bevorzugt weniger als 10 Vol.% an Komponenten enthält, welche keine Edukte, End- oder Zwischenprodukte der Umsetzung von A(COOR)ₓ₊₁ mit den Alkoholen R¹OH und R²OH sind. Unter diese Mengenangaben fällt nicht die Menge s₁ an dem Alkohol R¹OH. Unter diese Menge fällt jedoch jeder Alkohol, welcher nicht R¹OH oder R²OH ist. Zur Verbesserung der Steuerbarkeit der Zusammensetzung des aus dem erfindungsgemäßen Verfahrens resultierenden Estergemisches enthält die Reaktionsmischung während der Umsetzung mit Vorzug weniger als 0,5 Mol-Äquivalent, bevorzugt weniger als 0,1 Mol-Äquivalent, besonders bevorzugt weniger als 0,05 Mol-Äquivalent und insbesondere weniger als 0,01 Mol-Äquivalent an Alkoholen, welche keine Alkohole gemäß der Definition von R¹OH oder R²OH sind, wobei sich die Mol-Äquivalente auf die Gesamtheit aller in der Reaktionsmischung enthaltenen Alkohole (entsprechend 1 Mol-Äquivalent) beziehen.

Mittels des erfindungsgemäßen Verfahrens werden Estergemische erhalten, welche bevorzugt mindestens 5 Mol-%, besonders bevorzugt mindestens 10 Mol-%, dazu bevorzugt mindestens 15 Mol-% und insbesondere mindestens 20 Mol-% des/der Mischester(s) (Mol-% der Ester A(COOR¹)(COOR²)ₓ und A(COOR²)(COOR¹)ₓ zusammen), basierend auf der Gesamtheit der Ester A(COOR¹)(COOR¹)ₓ, A(COOR¹)(COOR²)ₓ, A(COOR²)(COOR¹)ₓ und A(COOR²)(COOR²)ₓ enthalten. Bevorzugt kann es zudem sein, wenn die Gesamtheit der Ester A(COOR¹)(COOR¹)ₓ, A(COOR¹)(COOR²)ₓ, A(COOR²)(COOR¹)ₓ und A(COOR²)(COOR²)ₓ den/die Mischester in einer Menge von mindestens 25 Mol-%, mit Vorzug dazu mindestens 30 Mol-%, mit besonderem Vorzug mindestens 35 Mol-%, weiter bevorzugt mindestens 40 Mol-%, und insbesondere mindestens 45 Mol-% enthält.

Zudem sind Verfahren bevorzugt, deren Produkt maximal 50 Mol-%, bevorzugt maximal 45 Mol-% und insbesondere maximal 40 Mol-% des/der Mischester(s) (Mol-% der Ester A(COOR¹)(COOR²)ₓ und A(COOR²)(COOR¹)ₓ zusammen) enthält, basierend auf der Gesamtheit der Ester A(COOR¹)(COOR¹)ₓ, A(COOR¹)(COOR²)ₓ, A(COOR²)(COOR¹)ₓ und A(COOR²)(COOR²)ₓ.

In bevorzugten Ausführungsformen des erfindungsgemäßen Verfahrens ermöglicht dieses die Bereitstellung von Estergemischen, in welchem das Molverhältnis der Ester A(COOR¹)(COOR¹)ₓ, A(COOR¹)(COOR²)ₓ, A(COOR²)(COOR¹)ₓ und A(COOR²)(COOR²)ₓ für x = 1 um weniger als 15 Punkte, vorzugsweise um weniger als 10 Punkte von dem statistisch ermittelten Erwartungswert abweicht, welcher sich ergibt, wenn man von einem vollständigen Einbau der Alkoholreste OR² ausgeht, wobei dieser Punkte-Wert der Summe aller Beträge der Differenzen zwischen statistischem Erwartungswert und tatsächlichem Mol-Anteil jedes einzelnen Esters im Estergemisch entspricht für den Fall, dass sich die Summe der Mol-Anteile der oben genannten Ester im Estergemisch zu 100 addiert.

Das erfindungsgemäße Verfahren wird vorzugsweise in Anwesenheit eines Katalysators oder mehrerer Katalysatoren, beispielsweise unter Verwendung von Brönstedt- oder Lewis-Säuren oder -Basen als Katalysator, durchgeführt. Als besonders geeignete Katalysatoren haben sich Schwefelsäure, Methansulfonscäure, p-Toluolsulfonsäure, Metalle oder deren Verbindungen erwiesen. Beispiele für besonders bevorzugte Metallkatalysatoren sind Zinnpulver, Zinn(II)oxid, Zinn(II)oxalat, Titansäureester wie Tetraisopropylorthotitanat oder Tetrabutylorthotitanat sowie Zirkoniumester wie Tetrabutylzirkonat sowie Natriummethanolat und Kaliummethanolat.

Das erfindungsgemäße Verfahren kann in dem Fachmann bekannten, typischen Veresterungsapparaturen unter üblichen Verfahrensbedingungen durchgeführt werden. Vorzugsweise findet das Verfahren bei Temperaturen bei oder oberhalb des Siedepunktes des Alkoholes R¹OH, des Siedepunktes von Wasser und/oder des Siedepunktes eines aus R¹OH und Wasser gebildeten Azeotropes statt, so dass die Überschussmenge s₁ des Alkohols R¹OH bei dem vorgegebenen Druck aus dem Reaktionsgemisch abdestilliert werden kann. Eine weitere Verbesserung der Steuerbarkeit der Zusammensetzung des aus dem erfindungsgemäßen Verfahren resultierenden Estergemisches wird erreicht, wenn das erfindungsgemäße Verfahren in einer Apparatur mit Kolonne durchgeführt wird. Diese gewährleistet vorzugsweise, dass während der Reaktion in der Gasphase befindlicher Alkohol R²OH möglichst quantitativ in das Reaktionsgefäß zurückgeführt wird. Unter dem Begriff "quantitativ" wird in diesem Zusammenhang zu mehr als 80 Mol-%, vorzugsweise zu mehr als 90 Mol-% und insbesondere zu mehr als 95 Mol-%, basierend auf der Menge des eingesetzten Alkohols R²OH, verstanden.

Bevorzugt wird das bei der Reaktion gebildete Wasser aus dem Reaktionsraum entfernt. Vorzugsweise dient dabei der Alkohol R¹OH dabei als Schleppmittel. Optional kann in dem erfindungsgemäßen Verfahren weiteres Schleppmittel, beispielsweise Cyclohexan, Toluol, Benzol oder Xylol eingesetzt werden.

Eine Verkürzung der für die Durchführung des erfindungsgemäßen Verfahrens benötigte Zeit wird möglich, wenn die beiden Alkohole R¹OH und R²OH nicht zeitgleich eingesetzt werden, sondern zumindest Teile des Alkohols R¹OH später als der Alkohol R²OH zu A(COOR)ₓ₊₁ hinzugesetzt wird. Bevorzugt werden deshalb zumindest Teile des Alkohols R¹OH später als der Alkohol R²OH mit A(COOH)ₓ₊₁ oder deren Derivaten zur Reaktion gebracht. Mit Vorzug wird A(COOR)ₓ₊₁ mit dem Alkohol R²OH und optional einem Katalysator sowie Teilen von R¹OH zum Sieden erhitzt und die verbleibenden Teile des Alkohols R¹OH werden erst zu einem späteren Zeitpunkt zu diesem Reaktionsgemisch gegeben.

Vorzugsweise werden während der Reaktion in regelmäßigen Abständen GC-Chromatogramme angefertigt oder die Säurezahl bestimmt, um den Fortschritt der Reaktion zu beobachten. Mit Vorzug wird die Reaktion durch Abkühlen und/oder Zerstörung des Katalysators, beispielsweise durch Zugabe von Wasser und/oder Base, abgebrochen, nachdem in den GC-Chromatogrammen der Restgehalt an eingesetzter A(COOR)ₓ₊₁ einen bestimmten Wert unterschreitet. Wird in dem erfindungsgemäßen Verfahren ein Derivat einer Säure A(COOH)ₓ₊₁, beispielsweise ein Dimethylester, eingesetzt, so kann als Grenzpunkt einer im Wesentlichen vollständig abgelaufenen Reaktion, der Gehalt der eingesetzten Komponente selbst oder aber der Gehalt eines Zwischenproduktes, beispielsweise im Fall eines eingesetzten Dimethylesters der Gehalt aller Monomethylester innerhalb des Reaktionsgemisches, vorzugsweise mittels GC, bestimmt werden. Mit Vorzug wird die Reaktion durch Abkühlen und/oder Zerrstörung des Katalysators abgebrochen, nachdem in den GC-Chromatogrammen ein Restgehalt an A(COOR)ₓ₊₁ oder an Zwischenprodukt, wie beispielsweise Monomethylester, von kleiner als 5,0 Flächen-%, bevorzugt von kleiner als 2,0 Flächen-% und insbesondere von kleiner 1,0 Flächen-%, bezogen auf die Gesamtfläche aller Ester im GC-Chromatogramm, festgestellt wird.

In einer besonders bevorzugten Ausführungsform wird in dem erfindungsgemäßen Verfahren ein Katalysator eingesetzt und dieser zerstört, nachdem im Reaktionsgemisch der Gehalt an R¹OH auf weniger als 15 Vol.-%, vorzugsweise auf weniger als 10 Vol.-% und insbesondere auf weniger als 5 Vol.-% - bezogen auf das Volumen des gesamten Reaktionsgemisches abgesenkt wurde. Mit besonderem Vorzug wird der Gehalt an R¹OH dabei auf weniger als 3 Vol.-% und insbesondere auf weniger als 1 Vol.-%, bezogen auf das Volumen des gesamten Reaktionsgemisches, vorzugsweise mittels Abdestillierens, abgesenkt. Eine besonders bevorzugte Ausführungsform des erfindungsgemäßen Verfahrens liegt vor, wenn in dem Verfahren ein Katalysator eingesetzt wird und im Reaktionsgemisch der Gehalt an R¹OH auf weniger als 20 Mol-%, vorzugsweise auf weniger als 15 Mol-%, bevorzugt auf weniger als 10 Mol-% und insbesondere auf weniger als 5 Mol-% - bezogen auf die Überschussmenge s₁ des Alkoholes R¹OH - abgesenkt wurde, bevor der Katalysator zerstört wird. Hierbei findet die Zerstörung des Katalysators vorzugsweise statt, nachdem mittels Reaktionskontrolle ein Reaktionsfortschritt von mindestens 90 % festgestellt wurde, beispielsweise indem in den Reaktionsfortschritt kontrollierenden GC-Chromatogrammen ein Restgehalt an eingesetztem A(COOR)ₓ₊₁ oder an Zwischenprodukt, wie beispielsweise Monomethylester, von kleiner als 5,0 Flächen-%, insbesondere von kleiner 1,0 Flächen-%, bezogen auf die Gesamtfläche aller Ester im GC-Chromatogramm, festgestellt wird oder indem eine bestimmte Säurezahl unterschritten wird. Durch das Absenken des Gehaltes an R¹OH wird eine verbesserte Steuerbarkeit des erfindungsgemäßen Verfahrens zu Estergemischen erreicht, deren Zusammensetzungen besonders geringe Abweichungen vom statistischen Erwartungswert aufweisen.

Nach Beenden der Reaktion wird das Reaktionsgemisch in üblicher Art und Weise aufgearbeitet.

Ein bevorzugter Gegenstand der vorliegenden Erfindung ist ein Verfahren zur Herstellung von Estergemischen umfassend A(COOR¹)(COOR¹)ₓ, A(COOR¹)(COOR²)ₓ, A(COOR²)(COOR¹)ₓ und A(COOR²)(COOR²)ₓ durch Umsetzung von A(COOR)ₓ₊₁, welche keine Estergruppen COOR mit einem Rest R enthalten, dessen Alkohol ROH einen höheren Siedepunkt bei einem bestimmten Druck aufweist als der Alkohol R¹OH des Restes R¹ bei demselben Druck, mit einer Menge (m₁ + s₁) R¹OH und einer Menge m₂ R²OH, in welchem
- das Reaktionsgemisch zum Sieden erhitzt wird,
- vorzugsweise die Reaktion abgebrochen wird, nachdem ein Reaktionsfortschritt von mindestens 90 % festgestellt wurde, beispielsweise indem die Säurezahl des Reaktionsgemisches einen Wert von 1,00 mg KOH pro g Reaktionsgemisch, insbesondere einen Wert von 0,50 mg KOH pro g Reaktionsgemisch unterschreitet oder in den Reaktionsfortschritt kontrollierenden GC-Chromatogrammen ein Restgehalt an einer während der Reaktion umgesetzten Komponente wie der A(COOR)ₓ₊₁ oder einem Zwischenprodukt von kleiner als 5,0 Flächen-%, insbesondere von kleiner 1,0 Flächen-%, bezogen auf die Gesamtfläche aller Ester im GC-Chromatogramm, festgestellt wird,
- vorzugsweise mindestens 0,8 Mol-Äquivalente, bevorzugt mindestens 0,9 Mol-Äquivalente und insbesondere mindestens 0,95 Mol-Äquivalente des in dem erfindungsgemäßen Verfahren eingesetzten Alkohols R²OH als Alkoholrest OR² in A(COOR)ₓ₊₁ eingeführt wird,
- vorzugsweise ein Katalysator eingesetzt und im Reaktionsgemisch der Gehalt an R¹OH auf weniger als 20 Mol-%, vorzugsweise auf weniger als 15 Mol-%, bevorzugt auf weniger als 10 Mol-% und insbesondere auf weniger als 5 Mol-% - bezogen auf die Überschussmenge s₁ des Alkoholes R¹OH - abgesenkt wurde, bevor der Katalysator zerstört wird,
- und wobei
   - A: für einen aromatischen, alicyclischen oder aliphatischen Rest steht,
   - x: 1 ist,
   - R¹ und R²: unabhängig voneinander für substituierte oder nichtsubstituierte Arylreste oder lineare oder verzweigte, substituierte oder nicht substituierte Alkylreste mit 3 bis 20 Kohlenstoffatomen stehen, wobei der Alkohol R¹OH einen niedrigeren Siedepunkt bei einem bestimmten Druck aufweist als der Alkohol R²OH bei demselben Druck,
   - m₁ und m₂: den Mol-Äquivalenten der in A(COOH)ₓ₊₁ oder deren Derivaten einzuführenden Alkoholresten OR¹ und OR² entspricht und
   - s₁: kleiner als m₁ + m₂ ist und insbesondere im Bereich des 0,05- bis 0,60-fachen von (m₁ + m₂) liegt.

Hierbei weicht vorzugsweise das Molverhältnis der Ester A(COOR¹)(COOR¹)ₓ, A(COOR¹)(COOR²)ₓ, A(COOR²)(COOR¹)ₓ und A(COOR²)(COOR²)ₓ für x = 1 um weniger als 15 Punkte, vorzugsweise um weniger als 10 Punkte von dem statistisch ermittelten Erwartungswert ab, welcher sich ergibt, wenn man von einem vollständigen Einbau der Alkoholreste OR² ausgeht, wobei dieser Punkte-Wert der Summe aller Beträge der Differenzen zwischen statistischem Erwartungswert und tatsächlichem Mol-Anteil jedes einzelnen Esters im Estergemisch entspricht für den Fall, dass sich die Summe der Mol-Anteile der oben genannten Ester im Estergemisch zu 100 addiert.

In einigen Ausführungsformen des erfindungsgemäßen Verfahrens wird ein Estergemisch hergestellt, in welchem die Ester hydrierbare Funktionalitäten wie zum Beispiel C=C-Doppelbindungen oder aromatische Reste aufweisen. Diese können - optional nach einem oder mehreren Aufarbeitungsschritten - in einem nachfolgenden Verfahrensschritt hydriert und die Ester so zu gesättigten (das heißt C-C-Mehrfachbindungs- oder Aromaten-freien) Verbindungen umgesetzt werden, welche vorzugsweise geeignet sind, als Weichmacher oder Weichmacherkomponente in Polymeren, beispielsweise PVC, eingesetzt zu werden. Bevorzugt handelt es sich in dem erfindungsgemäßen Verfahren um ein Estergemisch der Phthalsäure, der Terephthalsäure oder der Isophthalsäure und das Estergemisch wird in einem nachfolgenden Verfahrensschritt hydriert.

Ein weiterer bevorzugter Gegenstand der vorliegenden Erfindung ist ein Verfahren zur Herstellung von Estergemischen umfassend 1,2-, 1,3- oder 1,4-Ester der Cyclohexandicarbonsäure umfassend Cyclohexandicarbonsäuredipentylester, Cyclohexandicarbonsäurediisononylester und Cyclohexandicarbonsäurepentyl(isononyl)ester, durch Umsetzung von Terephthalsäure, Isophthalsäure oder Phthalsäure oder Derivaten dieser Säuren, welche keine Estergruppen COOR mit einem Rest R enthalten, dessen Alkohol ROH einen höheren Siedepunkt bei einem bestimmten Druck aufweist als Pentanol bei demselben Druck,
- mit einer Menge (m₁ + s₁) Pentanol und einer Menge m₂ Isononanol,
- wobei das Reaktionsgemisch zum Sieden erhitzt wird und ein Katalysator eingesetzt und im Reaktionsgemisch der Gehalt an Pentanol auf weniger als 20 Mol-%, vorzugsweise auf weniger als 15 Mol-%, bevorzugt auf weniger als 10 Mol-% und insbesondere auf weniger als 5 Mol-% - bezogen auf die Überschussmenge s₁ des Alkoholes R¹OH - abgesenkt wurde, bevor der Katalysator zerstört wird,
- das Estergemisch in einem nachfolgenden Verfahrensschritt einer Kernhydrierung unterzogen wird
- und wobei
   - m₁ und m₂: den Mol-Äquivalenten der in Terephthalsäure oder ihren Derivaten einzuführenden Alkoholresten von Pentanol und Isononanol entspricht und
   - s₁: größer als 0 ist und insbesondere im Bereich des 0,05- bis 0,60-fachen von (m₁ + m₂) liegt.

Ein anderer bevorzugter Gegenstand der vorliegenden Erfindung ist ein Verfahren zur Herstellung von Estergemischen umfassend 1,2-, 1,3- oder 1,4-Ester der Cyclohexandicarbonsäure umfassend Cyclohexandicarbonsäuredipentylester, Cyclohexandicarbonsäuredipropylheptylester und Cyclohexandicarbonsäure-pentyl(propylheptyl)ester, durch Umsetzung von Terephthalsäure, Isophthalsäure oder Phthalsäure oder Derivaten dieser Säuren, welche keine Estergruppen COOR mit einem Rest R enthalten, dessen Alkohol ROH einen höheren Siedepunkt bei einem bestimmten Druck aufweist als Pentanol bei demselben Druck,
- mit einer Menge (m₁ + s₁) Pentanol und einer Menge m₂ Propylheptanol,
- wobei das Reaktionsgemisch zum Sieden erhitzt wird und ein Katalysator eingesetzt und im Reaktionsgemisch der Gehalt an R¹OH auf weniger als 20 Mol-%, vorzugsweise auf weniger als 15 Mol-%, bevorzugt auf weniger als 10 Mol-% und insbesondere auf weniger als 5 Mol-% - bezogen auf die Überschussmenge s₁ des Alkoholes Pentanol - abgesenkt wurde, bevor der Katalysator zerstört wird,
- das Estergemisch in einem nachfolgenden Verfahrensschritt einer Kernhydrierung unterzogen wird
- und wobei
   - m₁ und m₂: den Mol-Äquivalenten der in Terephthalsäure oder ihren Derivaten einzuführenden Alkoholresten von Pentanol und Propylheptanol entspricht und
   - s₁: größer als 0 ist und insbesondere im Bereich des 0,05- bis 0,60-fachen von (m₁ + m₂) liegt.

Vorzugsweise sind bei den beiden obigen Ausführungsformen wiederum solche Pentyl- und Isononyl-Reste enthalten, wie weiter oben im Text definiert.

Ein weiterer bevorzugter Gegenstand der vorliegenden Erfindung ist ein Verfahren zur Herstellung von Estergemischen umfassend 1,2-, 1,3- oder 1,4-Ester der Cyclohexandicarbonsäure umfassend Cyclohexandicarbonsäurediisoheptylester, Cyclohexandicarbonsäuredi*iso*nonylester und Cyclohexandicarbonsäure-isoheptyl(isononyl)ester, durch Umsetzung von Terephthalsäure, Isophthalsäure oder Phthalsäure oder Derivaten dieser Säuren, welche keine Estergruppen COOR mit einem Rest R enthalten, dessen Alkohol ROH einen höheren Siedepunkt bei einem bestimmten Druck aufweist als Isoheptanol bei demselben Druck,
- mit einer Menge (m₁ + s₁) Isoheptanol und einer Menge m₂ Isononanol,
- wobei das Reaktionsgemisch zum Sieden erhitzt wird und ein Katalysator eingesetzt und im Reaktionsgemisch der Gehalt an R¹OH auf weniger als 20 Mol-%, vorzugsweise auf weniger als 15 Mol-%, bevorzugt auf weniger als 10 Mol-% und insbesondere auf weniger als 5 Mol-% - bezogen auf die Überschussmenge s₁ des Alkoholes Isoheptanol - abgesenkt wurde, bevor der Katalysator zerstört wird,
- das Estergemisch in einem nachfolgenden Verfahrensschritt einer Kernhydrierung unterzogen wird
- und wobei
   - m₁ und m₂: den Mol-Äquivalenten der in Terephthalsäure oder ihren Derivaten einzuführenden Alkoholresten von Isoheptanol und Isononanol entspricht und
   - s₁: größer als 0 ist und insbesondere im Bereich des 0,05- bis 0,60-fachen von (m₁ + m₂) liegt.

Weiterer Gegenstand der vorliegenden Erfindung ist ein Estergemisch, hergestellt nach dem beschriebenen erfindungsgemäßen Verfahren.

Insbesondere ist ein Gegenstand der vorliegenden Erfindung ein Estergemisch, welches nach einem der oben beschriebenen erfindungsgemäßen Verfahren hergestellt wurde und in welchem das Molverhältnis der Ester A(COOR¹)(COOR¹)ₓ, A(COOR¹)(COOR²)ₓ, A(COOR²)(COOR¹)ₓ und A(COOR²)(COOR²)ₓ für x = 1 um weniger als 15 Punkte, vorzugsweise um weniger als 10 Punkte vom statistisch ermittelten Erwartungswert abweicht, welcher sich ergibt, wenn man von einem vollständigen Einbau der Alkoholreste OR² ausgeht, wobei dieser Punkte-Wert der Summe aller Beträge der Differenzen zwischen statistischem Erwartungswert und tatsächlichem Mol-Anteil jedes einzelnen Esters im Estergemisch entspricht für den Fall, dass sich die Summe der Mol-Anteile der oben genannten Ester im Estergemisch zu 100 addiert.

Gegenstand der vorliegenden Erfindung ist zudem die Verwendung des erfindungsgemäßen Verfahrens zur Einstellung verarbeitungs- und/oder anwendungsrelevanter Eigenschaften eines Estergemisches durch Steuerung der Mengenverteilung der Ester im Estergemisch. Beispiele für die Beeinflussung einiger im Bereich der Weichmacher verarbeitungs- und anwendungsrelevanter Eigenschaften durch die Wahl des m₁:m₂-Verhältnisses sind oben im Text aufgeführt.

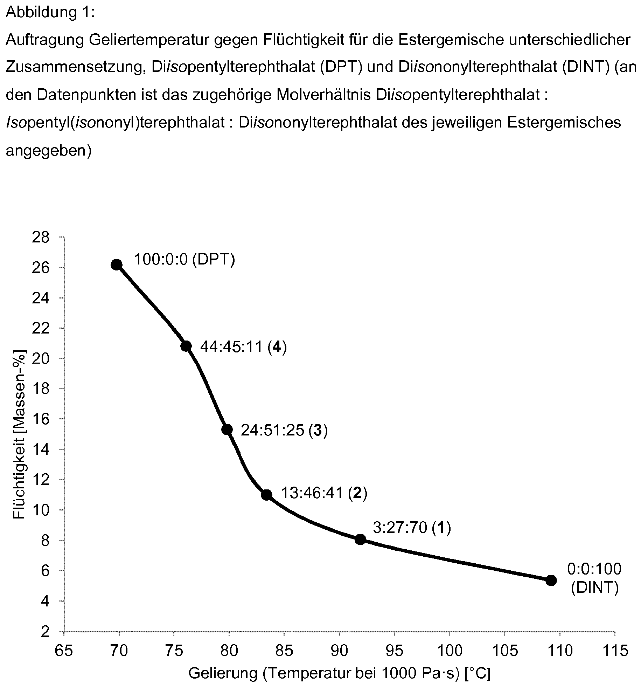

Besonders bevorzugter Gegenstand der vorliegenden Erfindung ist die Verwendung des erfindungsgemäßen Verfahrens zur Steuerung der Geliertemperatur eines Estergemisch-haltigen Plastisols und/oder der Steuerung der Flüchtigkeit eines Estergemisch-haltigen Prüfkörpers durch Steuerung der Mengenverteilung der Ester im Estergemisch. In der Abbildung 1 ist für erfindungsgemäße Estergemische mit unterschiedlicher Mengenverteilung der enthaltenen Ester die Geliertemperatur der Plastisole gegen die zugehörige Flüchtigkeit der dasselbe Estergemisch enthaltenden Folien aufgetragen. Aus dieser Abbildung wird ersichtlich, dass Produkte (Plastisole bzw. Folien) unterschiedlicher erfindungsgemäßer Gemische voneinander abweichende Geliertemperaturen und Flüchtigkeiten aufweisen und der Fachmann je nach der für die Verarbeitung und/oder Anwendung benötigten Geliertemperatur und Flüchtigkeit die geeignete Zusammensetzung eines Estergemisches auswählen kann. Selbiges gilt für andere Eigenschaften wie beispielsweise die unter anderem mittels des im experimentellen Teil beschriebenen Testes bestimmbare Verträglichkeit der Estergemische mit Polymeren, die die Weichmacher-Effizienz beschreibenden Shore-Härte, die Veränderung der Viskosität eines entsprechenden Plastisols nach Lagerung oder die Massenveränderung eines entsprechenden Plastisols bei Wasserlagerung. Das erfindungsgemäße Verfahren ermöglicht dem Fachmann nun, gezielt Estergemische mit den gewünschten Eigenschaften herzustellen, da mittels des erfindungsgemäßen Verfahrens gezielt die Mengenverteilung der Ester im Estergemisch eingestellt werden kann.

Das mittels des erfindungsgemäßen Verfahrens hergestellte Estergemisch wird vorzugsweise als Weichmacher oder als eine Komponente eines Gemisches mehrerer Polymer-weichmachender Verbindungen, welches hier ebenfalls als Weichmachergemisch bezeichnet werden soll, eingesetzt.

Ein Gegenstand der vorliegenden Erfindung ist weiterhin die Verwendung eines Estergemisches, welches gemäß dem erfindungsgemäßen Verfahren hergestellt wurde, als Weichmacher für Polymere.

Geeignete Polymere sind vorzugsweise ausgewählt aus der Gruppe, die gebildet wird durch Polyvinylchlorid (PVC), Homo- oder Copolymere auf Basis von Ethylen, Propylen, Butadien, Vinylacetat, Glycidylacrylat, Glycidylmethacrylat, Ethylacrylat, Butylacrylat oder Methacrylat mit Alkoxyresten von verzweigten oder unverzweigten Alkoholen mit einem bis zehn Kohlenstoffatome(n), Acrylnitril oder cyclischen Olefinen, Polyvinylidenchlorid (PVDC), Polyacrylaten, insbesondere Polymethylmethacrylat (PMMA), Polyalkylmethacrylat (PAMA), Polyharnstoffe, silylierte Polymere, Fluorpolymere, insbesondere Polyvinylidenfluorid (PVDF), Polytetrafluorethylen (PTFE), Polyvinylacetat (PVAc), Polyvinylalkohol (PVA), Polyvinylacetale, insbesondere Polyvinylbutyral (PVB), Polystyrolpolymere, insbesondere Polystyrol (PS), expandierbares Polystyrol (EPS), Acrylonitril-Styrol-Acrylat (ASA), Styrolacrylonitril (SAN), Acrylonitril-Butadien-Styrol (ABS), Styrol-Maleinsäureanhydrid-Copolymer (SMA), Styrol-Methacrylsäure-Copolymer, Polyolefine, insbesondere Polyethylen (PE) oder Polypropylen (PP), thermoplastische Polyolefine (TPO), Polyethylen-Vinylacetat (EVA), Polycarbonate, Polyethylenterephthalat (PET), Polybutylenterephthalat (PBT), Polyoxymethylen (POM), Polyamid (PA), Polyethylenglykol (PEG), Polyurethan (PU), Thermoplastisches Polyurethan (TPU), Polysulfide (PSu), Biopolymere, insbesondere Polymilchsäure (PLA), Polyhydroxybutyral (PHB), Polyhydroxyvaleriansäure (PHV), Polyester, Stärke, Cellulose und CelluloseDerivate, insbesondere Nitrocellulose (NC), Ethylcellulose (EC), Celluloseacetat (CA), Cellulose-Acetat/Butyrat (CAB), Gummi und Silikone.

Bevorzugte Polymere sind Polyvinylchlorid, Co-Polymere von Vinylchlorid mit Vinylacetat oder mit Butylacrylat, Polyalkylmethacrylat (PAMA), Polyvinylbutyral (PVB), Polyurethan, Polysulfide, Polymilchsäure (PLA), Polyhydroxybutyral (PHB) und Nitrocellulose.

Besonders bevorzugt ist die Verwendung eines Estergemisches, welches gemäß dem erfindungsgemäßen Verfahren hergestellt wurde, als Weichmacher für PVC.

Vorzugsweise wird das mittels des erfindungsgemäßen Verfahrens hergestellte Estergemisch als Weichmacher in Klebstoffen, Dichtungsmassen, Beschichtungsmassen, Lacken, Farben, Plastisolen, Schäumen, Kunstleder, Fußbodenbelägen (z.B. Deckschicht), Dachbahnen, Unterbodenschutz, Gewebebeschichtungen, Kabeln, Drahtisolierungen, Schläuchen, Extrusionsartikeln, Folien, im Automobilinnenbereich, in Tapeten, Tinten, Spielzeug, Kontaktfolien, Lebensmittelverpackungen oder medizinischen Artikeln, beispielsweise Schläuchen oder Blutbeuteln, verwendet.

Weiterer Gegenstand der vorliegenden Erfindung ist ein Mittel enthaltend ein Estergemisch, welches gemäß dem erfindungsgemäßen Verfahren hergestellt wurde, sowie ein oder mehrere Polymere aus der Gruppe die gebildet wird von Polyvinylchlorid, Co-Polymere von Vinylchlorid mit Vinylacetat oder mit Butylacrylat, Polyalkylmethacrylat (PAMA), Polyvinylbutyral (PVB), Polyurethan, Polysulfide, Polymilchsäure (PLA), Polyhydroxybutyral (PHB) und Nitrocellulose.

Bezogen auf 100 Massenteile Polymer enthalten bevorzugte Mittel von 5 bis 200, bevorzugt von 10 bis 150 Massenteile an Weichmacher.

Bevorzugt ist die Verwendung des erfindungsgemäßen Estergemisches als Weichmacher für Polyvinylchlorid und dementsprechend sind Mittel, welche das erfindungsgemäße Estergemisch und PVC enthalten, besonders bevorzugt.

Bevorzugt ist das Polymer ein Suspensions-, Masse-, Mikrosuspensions- oder Emulsions-PVC.

Bevorzugte erfindungsgemäße Mittel können neben dem erfindungsgemäßen Estergemisch mindestens eine weitere Polymer-weichmachende Verbindung, also einen weiteren Weichmacher, enthalten. Eine besonders bevorzugte Ausführungsform des erfindungsgemäßen Mittels liegt vor, wenn dieses weniger als 5 Massen-% und insbesondere weniger als 0,5 Massen-% Phthalat-haltige Verbindungen enthält. Die weiteren Weichmacher werden vorzugsweise ausgewählt aus der Gruppe der Adipate, Benzoate, beispielsweise Monobenzoate oder Glycoldibenzoate, chlorierten Kohlenwasserstoffe, Citrate, Cyclohexandicarboxylate, epoxidierten Fettsäureester, epoxidierten Pflanzenöle, epoxidierten acylierten Glyceride, Furandicarboxylate, Phosphate, Phthalate (vorzugsweise in möglichst geringen Mengen), Succinate, Sulfonamide, Sulfonate, Terephthalate, Trimellitate oder oligomeren oder polymeren Ester auf Basis von Adipin-, Bernstein- oder Sebacinsäure. Besonders bevorzugt sind Alkylbenzoate, Dialkyladipate, Glycerinester, Citronensäuretrialkylester, acylierte Citronensäuretrialkylester, Trialkyltrimellitate, Glykoldibenzoate, Dialkylterephthalate, Ester der Furandicarbonsäure, Dialkanoylester von Dianhydrohexitolen (z.B. Isosorbit) und Dialkylester der 1,2-, 1,3- oder 1,4-Cyclohexandicarbonsäure.

In einer Ausführungsform enthält das erfindungsgemäße Mittel neben dem erfindungsgemäßen Estergemisch weniger als 20 Massen-%, weniger als 10 Massen-% oder keinen weiteren Weichmacher, wobei die Massen-% auf der Gesamtmasse des Mittels basieren.

Erfindungsgemäße Mittel enthalten vorzugsweise neben dem Polymer oder einer Mischung mehrerer Polymere und dem erfindungsgemäßen Estergemisch ein oder mehrere Additive aus der Gruppe der Thermostabilisatoren, Füllstoffe, Pigmente, Treibmittel, Biozide, UV- und Licht-Stabilisatoren, Co-Stabilisatoren, Antioxidantien, Viskositätsregler, Entlüfter, Haftvermittler, Gleitmittel und Färbemittel.

Die erfindungsgemäßen Mittel können in Klebstoffen, Dichtungsmassen, Beschichtungsmassen, Lacken, Farben, Plastisolen, Schäumen, Kunstleder, Fußbodenbelägen (z.B. Deckschicht), Dachbahnen, Unterbodenschutz, Gewebebeschichtungen, Kabeln, Drahtisolierungen, Schläuchen, Extrusionsartikeln, Folien, im Automobilinnenbereich, in Tapeten, Tinten, Spielzeug, Kontaktfolien, Lebensmittelverpackungen oder medizinischen Artikeln, beispielsweise Schläuchen oder Blutbeuteln, eingesetzt werden.

### Experimenteller Teil:

### Siedebereich der Alkohole:

Die in den Beispielen eingesetzten beziehungsweise zur Synthese der in den Beispielen eingesetzen Ester verwendeten Alkohole wiesen folgende Siedebereiche auf: Isononanol (Evonik Industries AG, Reinheit > 99 %): 205 bis 215 °C bei 1013 hPa; Isopentanol (Gemisch n-Pentanol (Sigma Aldrich, Reinheit > 99 %) und 2-Methylbutanol (Sigma Aldrich, Reinheit > 99 %) im Molverhältnis 1:1): 129 bis 138 °C bei 1013 hPa

### Säurezahl:

Die Säurezahl wurde in Anlehnung an DIN EN ISO 2114 bestimmt.

### GC-Analysen:

Die GC- Analyse erfolgte mit folgenden Parametern:
Kapillarsäule: 30 m DB5; 0,25 mm ID; 0,25 µm Film
Trägergas: Helium
Säulenvordruck: 80 kPa
Split: ca. 23,8 ml/min
Ofentemperaturprogramm (Dauer: 51 min): 50 °C (für 1 min), Aufheizen mit 7,5 °C/min auf 350 °C (Temperatur halten für 1 min)
Injektor: 350 °C
Detektor (FID): 400 °C
Injektionsvolumen: 1,0 µl

Die Identifizierung der Komponenten im Chromatogramm der Probe erfolgte mittels einer Vergleichslösung der relevanten Ester. Im Anschluss erfolgte eine Normierung der Signale im Chromatogramm der Probe auf 100 Flächen-%. Die Stoffmengenverhältnisse wurden in hinreichender Näherung aus den Flächenverhältnissen der einzelnen Signale bestimmt.

Die Reinheit wurde über den Anteil der Produktsignale an den Gesamtflächen im Chromatogramm bestimmt.

Diisononylterephthalat (DINT) und Diisopentylterephthalat (DPT) wurden wie im Stand der Technik bekannt mittels Veresterung von Dimethylterephthalat mit den oben angegebenen Alkoholen Isononanol bzw. Isopentanol hergestellt.

### Beispiel 1 (erfindungsgemäß):

Umesterung von Dimethylterephthalat mit Isopentanol und Isononanol (1,0 : 18,5 : 80,5)

In eine Umesterungsapparatur umfassend Rührkolben mit Rührer, Tauchrohr, Thermometer und 20 cm Raschigringkolonne mit aufgesetztem Destillationskopf wurden Dimethylterephthalat (Sigma Aldrich, Reinheit > 99 %) (m_{b}) und die Alkohole R¹OH (m₁ + s₁) und R²OH (m₂) eingefüllt. Die Apparatur wurde mindestens eine Stunde mit Stickstoff (6 l/h) über das Tauchrohr gespült. Anschließend wurden 0,25 Gew.-% Tetra-n-Butyltitanat (Sigma Aldrich, Reinheit > 97 %) bezogen auf die Masse des Terephthalsäureesters hinzugegeben. Das Gemisch wurde anschließend zum Sieden erhitzt und leichtsiedende Komponenten abdestilliert. Bei sprunghaftem Anstieg der Kopftemperatur wurde die Destillation durch Verschluss des Ablaufhahns unterbrochen und die Reaktion bis zur Einstellung einer stabilen Siedetemperatur am Rückfluss belassen. Während der Reaktion stieg die Sumpftemperatur von T¹ auf T² an. Während der Reaktion wurden stündlich GC-Chromatogramme angefertigt. Sobald in diesen ein Restgehalt an Monomethylester von kleiner als 0,5 Flächen-% bezogen auf die Gesamtfläche aller Ester im GC-Chromatogramm festgestellt wurde, wurden die verbliebenen leichtflüchtigen Komponenten des Reaktionsgemisches unter Vakuum (ca. 1 mbar) bei einer Sumpftemperatur von T³ abdestilliert, so dass der Restgehalt am Alkohol R¹OH unterhalb von 5 Mol-% - bezogen auf die Überschussmenge s₁ des Alkohols R¹OH (gemäß GC) - betrug. Anschließend wurde der Kolbeninhalt bei ausgeschalteter Heizung durch Einleiten von Stickstoff bei 20 mbar bis auf ca. 80 °C abgekühlt. Vom Kolbeninhalt wurde die Säurezahl bestimmt. Entsprechend des Ergebnisses wurde das Reaktionsmedium durch langsames Zutropfen der dreifachen stöchiometrischen Menge an Base (10 %-ige wässrige NaOH-Lösung) neutralisiert und unter Einleitung von Stickstoff (6 I/h) bei 80 °C für 15 min gerührt. Anschließend wurde der Ansatz langsam vom Umgebungsdruck bis auf ca. 1 mbar evakuiert, woraufhin auf ca. 120 °C aufgeheizt wurde und bei konstanter Temperatur restliche flüchtige Bestandteile mithilfe einer Stickstoffeinleitung abgetrennt wurde. Der Stickstoffstrom wird so eingestellt, dass der Druck nicht über 20 mbar stieg. Sobald der Restalkoholgehalt entsprechend GC-Analyse kleiner als 0,025 Flächen-% war, wurde die Heizung abgestellt und unter Vakuum und Einleiten von Stickstoff auf 80 °C abgekühlt. Bei dieser Temperatur wurde das Produkt über einen Büchnertrichter mit Filterpapier und vorgepresstem Filterkuchen aus Filterhilfsmittel (Perlite Typ D14) mittels Vakuum in eine Saugflasche filtriert. An dem Filtrat wurde eine GC-Analyse durchgeführt, an Hand derer die Reinheit (R) und die Zusammensetzung des Produkts analysiert wurde.

**Tabelle 2: gemessene und berechnete Werte zum Beispiel 1**

| | |
|---|---|
| R¹OH = Isopentanol | m₁ + s₁ = 308,5 g |
| Stoffmenge R¹OH = Stoffmenge R¹-Äquivalente (C₅) | 3,5 mol |
| R²OH = *Iso*nonanol | m₂ = 1296 g |
| Stoffmenge R²OH = Stoffmenge R²-Äquivalente (C₉) | 9 mol |
| Dimethylterephthalat | m_{b} = 970 g |
| Stoffmenge | 5 mol |
| → Stoffmenge Esterfunktionen | 10 mol |
| erwarteter Molanteil der R¹-Funktionen an R¹ und R²-Funktionen im Estergemisch (bei vollständigem Einbau von R²) | 10 mol - 9 mol = 1 mol |
| | entsprechend 10 Mol-% |
| Verhältnis m₁ : m₂ | 0,1 : 0,9 |
| s₁ | 3,5 mol - (10,0 mol - 9,0 mol) = 2,5 mol |
| T¹ → T² (Sumpftemperatur Anfang -> Ende) | 131 °C → 200 °C |
| T³ | 200 °C |
| R (Reinheit) | > 99,9 % |
| Statistische Erwartungswerte für vollständigen Einbau von C₉: | **1,0 : 18,0 : 81,0** |
| (C₅/C₅) : (C₅/C₉) : (C₉/C₉) | (erwarteter C₅-Anteil: 10 %) |
| Zusammensetzung gemäß GC | **1,0 : 18,5 : 80,5** (C₅-Anteil: 10,3 %) |
| (C₅/C₅) : (C₅/C₉) : (C₉/C₉) | Abweichung: |
| | \|1-1\| + \|18,5-18,0\| + 180,5-81,01 = 1 |

Es wurden 1902 g aufgearbeitetes Estergemisch erhalten.

### Beispiel 2 (erfindungsgemäß):

Umesterung von Dimethylterephthalat mit Isopentanol und Isononanol (0 : 12 : 87)

Das Beispiel 2 wurde ausgeführt wie für das Beispiel 1 beschrieben.

**Tabelle 3: gemessene und berechnete Werte zum Beispiel 2**

| | |
|---|---|
| R¹OH = Isopentanol | m₁ + s₁ = 264,5 g |
| Stoffmenge R¹OH = Stoffmenge R¹-Äquivalente (C₅) | 3 mol |
| R²OH = Isononanol | m₂ = 1368 g |
| Stoffmenge R²OH = Stoffmenge R²-Äquivalente (C₉) | 9,5 mol |
| Dimethylterephthalat | m_{b} = 970 g |
| Stoffmenge | 5 mol |
| → Stoffmenge Esterfunktionen | 10 mol |
| Erwarteter Molanteil der R¹-Funktionen an R¹ und R²-Funktionen im Estergemisch (bei vollständigem Einbau von R²) | 10 mol - 9,5 mol = 0,5 mol |
| | entsprechend 5 Mol-% |
| Verhältnis m₁ : m₂ | 0,05 : 0,95 |
| s₁ | 3 mol - (10 mol - 9,5 mol) = 2,5 mol |
| T¹ → T² (Sumpftemperatur Anfang -> Ende) | 138 °C → 200 °C |
| T³ | 200 °C |
| R (Reinheit) | 99,7 % |
| Statistische Erwartungswerte für vollständigen Einbau von C₉: | **0,25** : **9,5** : **90,25** |
| (C₅/C₅) : (C₅/C₉) : (C₉/C₉) | (erwarteter Cs-Anteil: 5,0 %) |
| Zusammensetzung gemäß GC | **0,4 : 12,4 : 87,2** (C₅-Anteil: 6,6 %) |
| (C₅/C₅) : (C₅/C₉) : (C₉/C₉) | Abweichung: |
| | \|0,4-0,25\| + \|12,4-9,5\| + \|87,2-90,25\| = 6,1 |

Es wurden 1966 g aufgearbeitetes Estergemisch erhalten.

### Beispiel 3 (erfindungsgemäß):

Umesterung von Dimethylterephthalat mit Isopentanol und Isononanol (13 : 46 : 41)

Das Beispiel 3 wurde ausgeführt wie für das Beispiel 1 beschrieben.

**Tabelle 4: gemessene und berechnete Werte zum Beispiel 3**

| | |
|---|---|
| R¹OH = Isopentanol | m₁ + s₁ = 441 g |
| Stoffmenge R¹OH = Stoffmenge R¹-Äquivalente (C₅) | 5 mol |
| R²OH = Isononanol | m₂ = 720 g |
| Stoffmenge R²OH = Stoffmenge R²-Äquivalente (C₉) | 5 mol |
| Dimethylterephthalat | m_{b} = 776 g |
| Stoffmenge | 4 mol |
| → Stoffmenge Esterfunktionen | 8 mol |
| Erwarteter Molanteil der R¹-Funktionen an R¹ und R²-Funktionen im Estergemisch (bei vollständigem Einbau von R²) | 8 mol - 5 mol = 3 mol |
| | entsprechend 37,5 Mol-% |
| Verhältnis m₁ : m₂ | 3 : 5 entsprechend 0,375 : 0,625 |
| s₁ | 5 mol - (8 mol - 5 mol) = 2 mol |
| T¹ → T² (Sumpftemperatur Anfang → Ende) | 139 °C → 215 °C |
| T³ | 190 °C |
| R (Reinheit) | 99,7 % |
| Statistische Erwartungswerte für vollständigen Einbau von C₉: | **14,1** : **46,8** : **39,1** |
| (C₅/C₅) : (C₅/C₉) : (C₉/C₉) | (erwarteter C₅-Anteil: 37,5 %) |
| Zusammensetzung gemäß GC | **13,4 : 45,8 : 40,8** (C₅-Anteil: 36 %) |
| (C₅/C₅) : (C₅/C₉) : (C₉/C₉) | Abweichung: |
| | \|14,1-13,4\| + \|46,8-45,8\| + \|39,1-40,8\| = 3,4 |

Es wurden 1270 g aufgearbeitetes Estergemisch **13:46:41** erhalten.

### Beispiel 4 (erfindungsgemäß):

Umesterung von Dimethylterephthalat mit *Iso*pentanol und *Iso*nonanol (24 : 51 : 25)

Das Beispiel 4 wurde ausgeführt wie für das Beispiel 1 beschrieben.

**Tabelle 5: gemessene und berechnete Werte zum Beispiel 4**

| | |
|---|---|
| R¹OH = *Iso*pentanol | m₁ + s₁ = 705 g |
| Stoffmenge R¹OH = Stoffmenge R¹-Äquivalente (C₅) | 8 mol |
| R²OH = *Iso*nonanol | m₂ = 576 g |
| Stoffmenge R²OH = Stoffmenge R²-Äquivalente (C₉) | 4 mol |
| Dimethylterephthalat | m_{b} = 776 g |
| Stoffmenge | 4 mol |
| → Stoffmenge Esterfunktionen | 8 mol |
| Erwarteter Molanteil der R¹-Funktionen an R¹ und R²-Funktionen im Estergemisch (bei vollständigem Einbau von R²) | 8 mol - 4 mol = 4 mol |
| | entsprechend 50 Mol-% |
| Verhältnis m₁ : m₂ | 0,5 : 0,5 |
| s₁ | 8 mol - (8 mol - 4 mol) = 4 mol |
| T¹ → T² (Sumpftemperatur Anfang → Ende) | 123 °C → 174 °C |
| T³ | 180 °C |
| R (Reinheit) | 99,9 % |
| Statistische Erwartungswerte für vollständigen Einbau von C₉: | **25,0 : 50,0 : 25,0** |
| (C₅/C₅) : (C₅/C₉) : (C₉/C₉) | (erwarteter C₅-Anteil: 50 %) |
| Zusammensetzung gemäß GC | **24,0 : 50,5 : 25,5** (C₅-Anteil: 49,5 %) |
| (C₅/C₅) : (C₅/C₉) : (C₉/C₉) | Abweichung: |
| | \|25,0-24,0\| + \|50,0-50,5\| + \|25-25,5\| = 2 |

Es wurden 1254 g aufgearbeitetes Estergemisch **24:51:25** erhalten.

### Beispiel 5 (erfindungsgemäß):

Umesterung von Dimethylterephthalat mit Butanol und 2-Ethylhexanol (3 : 27 : 70)

Das Beispiel 5 wurde ausgeführt wie für das Beispiel 1 beschrieben.

**Tabelle 6: gemessene und berechnete Werte zum Beispiel 5**

| | |
|---|---|
| R¹OH = *n*-Butanol | m₁ + s₁ = 154 g |
| (Sigma Aldrich, Reinheit ≥ 99,4 %) | |
| Stoffmenge R¹OH = Stoffmenge R¹-Äquivalente (C₅) | 2,1 mol |
| R²OH = 2-Ethylhexanol | m₂ = 543 g |
| (Sigma Aldrich, Reinheit ≥ 99 %) | |
| Stoffmenge R²OH = Stoffmenge R²-Äquivalente (C₉) | 4,2 mol |
| Dimethylterephthalat | m_{b} = 485 g |
| Stoffmenge | 2,5 mol |
| → Stoffmenge Esterfunktionen | 5 mol |
| Erwarteter Molanteil der R¹-Funktionen an R¹ und R²-Funktionen im Estergemisch (bei vollständigem Einbau von R²) | 5 mol - 4,2 mol = 0,8 mol |
| | entsprechend 16 Mol-% |
| Verhältnis m₁ : m₂ | 0,8 : 4,2 entsprechend 0,16 : 0,84 |
| s₁ | 2,1 mol - (5 mol - 4,2 mol) = 1,3 mol |
| T¹ → T² (Sumpftemperatur Anfang -> Ende) | 123 °C → 215 °C |
| T³ | 160 °C |
| R (Reinheit) | > 99,9 % |
| Statistische Erwartungswerte für vollständigen Einbau von C₉: | **2,6** : **26,9** : **70,5** |
| (C₄/C₄) : (C₄/C₈) : (C₈/C₈) | (erwarteter C₄-Anteil: 16 %) |
| Zusammensetzung gemäß GC | **2,5** : **27,3** : **70,2** (C₄-Anteil: 16 %) |
| (C₄/C₄) : (C₄/C₈) : (C₈/C₈) | Abweichung: |
| | \|2,5-2,6\| + \|27,3-26,9\| + \|70,2-70,5\| = 0,8 |

Es wurden 920 g aufgearbeitetes Estergemisch erhalten.

### Beispiel 6 (erfindungsgemäß):

Umesterung von Dimethylterephthalat mit Butanol und 2-Ethylhexanol (11 : 45 : 44)

Das Beispiel 6 wurde ausgeführt wie für das Beispiel 1 beschrieben.

**Tabelle 7: gemessene und berechnete Werte zum Beispiel 6**

| | |
|---|---|
| R¹OH = *n*-Butanol | m₁ + s₁ = 232 g |
| (Sigma Aldrich, Reinheit ≥ 99,4 %) | |
| Stoffmenge R¹OH = Stoffmenge R¹-Äquivalente (C₅) | 3,1 mol |
| R²OH = 2-Ethylhexanol | m₂= 407 g |
| (Sigma Aldrich, Reinheit ≥ 99 %) | |
| Stoffmenge R²OH = Stoffmenge R²-Äquivalente (C₉) | 3,1 mol |
| Dimethylterephthalat | m_{b} = 485 g |
| Stoffmenge | 2,5 mol |
| → Stoffmenge Esterfunktionen | 5 mol |
| Erwarteter Molanteil der R¹-Funktionen an R¹ und R²-Funktionen im Estergemisch (bei vollständigem Einbau von R²) | 5 mol - 3,1 mol = 1,9 mol |
| | entsprechend 38 Mol-% |
| Verhältnis m₁ : m₂ | 1,9 : 3,1 entsprechend 0,38 : 0,62 |
| s₁ | 3,1 mol - (5 mol - 3,1 mol) = 1,2 mol |
| T¹ → T² (Sumpftemperatur Anfang -> Ende) | 123 °C → 200 °C |
| T³ | 160 °C |
| R (Reinheit) | 99,6 % |
| Statistische Erwartungswerte für vollständigen Einbau von C₉: | **14,4 : 47,1** : **38,5** |
| (C₄/C₄) : (C₄/C₈) : (C₈/C₈) | (erwarteter C₄-Anteil: 38 %) |
| Zusammensetzung gemäß GC | **10,8 : 45,0 : 44,1** (C₄-Anteil: 33 %) |
| (C₄/C₄) : (C₄/C₈) : (C₈/C₈) | Abweichung: |
| | \|14,4-10,8\| + \|45,0-47,1\| + \|44,1-38,5\| = 11,3 |

Es wurden 850 g aufgearbeitetes Estergemisch erhalten.

### Beispiel 7 (erfindungsgemäß):

Umesterung von Dimethylterephthalat mit Isopentanol und Isononanol (44 : 45 : 11)

In einer Umesterungsapparatur umfassend Rührkolben mit Rührer, Tauchrohr, Thermometer und 20 cm Raschigringkolonne mit aufgesetztem Destillationskopf wurde Dimethylterephthalat (Sigma Aldrich, Reinheit > 99 %) (m_{b}) vorgelegt und in dem Alkohol R²OH (m₂) suspendiert. Die Apparatur wurde mindestens eine Stunde mit Stickstoff (6 l/h) über das Tauchrohr gespült. Anschließend wurden 0,25 Gew.-% Tetra-n-Butyltitanat (Sigma Aldrich, Reinheit > 97 %) bezogen auf die Masse des Terephthalsäureesters hinzugegeben. Zur Homogenisierung des Reaktionsgemisches wurden 300 ml des Alkohols R¹OH hinzugegeben. Die Reaktionsmischung wurde anschließend langsam unter Rühren aufgeheizt. Reaktionsbeginn war bei T¹ Sumpftemperatur und bei 62 °C Kopftemperatur. Ab diesem Zeitpunkt fiel Methanol an, welches kontinuierlich unter leichtem Rückfluss über den Destillationskopf aus der Reaktion entfernt wurde. Bei sprunghaftem Anstieg der Kopftemperatur wurde die Destillation durch Verschluss des Ablaufhahns unterbrochen und die Reaktion bis zur Einstellung einer stabilen Siedetemperatur am Rückfluss belassen. Sobald trotz Steigerung der Sumpftemperatur kein Destillat mehr anfiel, wurde die Restmenge (m₁ + s₁ - 300 ml) von Alkohol R¹OH mit einer solchen Zugaberate hinzugegeben, dass die Sumpftemperatur nicht unter 150 °C sank. Die Reaktion wurde anschließend entlang der Siedetemperatur des Gemisches kontinuierlich bis T² erhitzt, so dass ein stetiges Destillat gewonnen werden konnte. Erneut wurde bei sprunghaftem Anstiegs der Kopftemperatur die Destillation durch Verschluss des Ablaufhahns unterbrochen und die Reaktion bis zur Einstellung einer stabilen Siedetemperatur am Rückfluss belassen. Sobald trotz Steigerung der Sumpftemperatur kein Destillat mehr anfiel und mittels GC-Analyse ein Restgehalt an Monomethylester von kleiner als 0,5 Flächen-% bezogen auf die Gesamtfläche aller Ester festgestellt wurde, wurden die verbleibenden leichtflüchtigen Komponenten bei T³ durch Anlegen von Vakuum direkt abdestilliert, so dass der Restgehalt am Alkohol R¹OH unterhalb von 5 Mol-% - bezogen auf die Überschussmenge s₁ des Alkohols R¹OH (gemäß GC) - betrug. Dann wurde der Kolbeninhalt bei ausgeschalteter Heizung durch Einleiten von Stickstoff bis auf ca. 80 °C abgekühlt. Vom Kolbeninhalt wurde die Säurezahl bestimmt. Entsprechend des Ergebnisses wurde das Reaktionsmedium durch langsames Zutropfen der dreifachen stöchiometrischen Menge an Base (10 %-ige wässrige NaOH-Lösung) neutralisiert und unter Einleitung von Stickstoff (6 I/h) bei 80 °C für 15 min gerührt. Anschließend wurde der Ansatz langsam vom Umgebungsdruck bis auf ca. 1 mbar evakuiert, woraufhin auf ca. 120 °C aufgeheizt wurde und bei konstanter Temperatur restliche flüchtige Bestandteile mithilfe einer Stickstoffeinleitung abgetrennt wurden. Der Stickstoffstrom wurde so eingestellt, dass der Druck nicht über 20 mbar stieg. Sobald der Restalkoholgehalt entsprechend GC-Analyse kleiner als 0,025 Flächen-% war, wurde die Heizung abgestellt und unter Vakuum und Einleiten von Stickstoff auf 80 °C abgekühlt. Bei dieser Temperatur wurde das Produkt über einen Büchnertrichter mit Filterpapier und vorgepresstem Filterkuchen aus Filterhilfsmittel (Perlite Typ D14) mittels Vakuum in eine Saugflasche filtriert. An dem Filtrat wurde eine GC-Analyse durchgeführt, an Hand derer die Reinheit (R) und die Zusammensetzung des Produkts analysiert wurde.

**Tabelle 8: gemessene und berechnete Werte zum Beispiel 7**

| | |
|---|---|
| R¹OH = *Iso*pentanol | m₁ + s₁ = 846 g |
| Stoffmenge R¹OH = Stoffmenge R¹-Äquivalente (C₅) | 9,6 mol |
| R²OH = *Iso*nonanol | m₂= 346 g |
| Stoffmenge R²OH = Stoffmenge R²-Äquivalente (C₉) | 2,4 mol |
| B = Dimethylterephthalat | m_{b} = 776 g |
| Stoffmenge | 4 mol |
| → Stoffmenge Esterfunktionen | 8 mol |
| Erwarteter Molanteil derR¹-Funktionen an R¹ und R²-Funktionen im Estergemisch (bei vollständigem Einbau von R²) | 8 mol - 2,4 mol = 5,6 mol |
| | entsprechend 70 Mol-% |
| Verhältnis m₁ : m₂ | 5,6 : 2,4 entsprechend 0,7 : 0,3 |
| s₁ | 9,6 mol - (8 mol - 2,4 mol) = 4 mol |
| T¹ → T² (Sumpftemperatur Anfang -> Ende) | 123 °C → 166 °C |
| T³ | 190 °C |
| R (Reinheit) | 99,8 % |
| Statistische Erwartungswerte für vollständigen Einbau von C₉: | **49,0** : **42,0** : **9,0** |
| (C₅/C₅) : (C₅/C₉) : (C₉/C₉) | (erwarteter C₅-Anteil: 70 %) |
| Zusammensetzung gemäß GC | **44,3** : **45,0** : **10,7** (C₅-Anteil: 66,8 %) |
| (C₅/C₅) : (C₅/C₉) : (C₉/C₉) | Abweichung: |
| | \|49,0-44,3\|) + \|42,0-45,0\| + \|9,0-10,7\| = 9,4 |

Es wurden 1174 g aufgearbeitetes Estergemisch **44:45:11** erhalten.

### Beispiel 8 (erfindungsgemäß):

Umesterung von Dimethylterephthalat mit Isopentanol und Isononanol (3 : 27 : 70)

Das Beispiel 8 wurde ausgeführt wie für das Beispiel 1 beschrieben, wobei Dimethylterephthalat mit Isopentanol und Isononanol eingesetzt wurde. Es wurde ein Estergemisch mit einer Zusammensetzung (C₅/C₅) : (C₅/C₉) : (C₉/C₉) von **3** : **27** : **70** gemäß GC erhalten.

### Beispiel 9 (nicht erfindungsgemäß):

Veresterung von Bernsteinsäure mit Isopentanol und Isononanol (27 : 51 : 22)

In eine Apparatur umfassend Rührkolben mit Rührer, Tauchrohr, Wasserabscheider mit aufgesetztem Intensivkühler, Thermometer und 20 cm Raschigringkolonne wurden die Säure oder das Säurederivat B (m_{b}) und die Alkohole R¹OH (m₁ + s₁) und R²OH (m₂) eingefüllt. Die Apparatur wurde mindestens eine Stunde mit Stickstoff (6 l/h) über das Tauchrohr gespült. Anschließend werden 0,25 Gew.-% Tetra-n-Butyltitanat (Sigma Aldrich, Reinheit > 97 %) bezogen auf die Masse der Säure bzw. des Säurederivates hinzugegeben. Die Reaktion wurde anschließend entlang der Siedetemperatur von T¹ auf T² erhitzt und dabei Wasser mittels des Wasserabscheiders aus dem System entfernt. Sobald bei maximaler Sumpftemperatur T² kein Rückfluss mehr aufrechterhalten werden konnte, wurde Cyclohexan (m_{Sch}) als Schleppmittel hinzugegeben. Sobald die stöchiometrische Menge an Wasser aus der Reaktion entfernt worden war (reale Menge = m_{d1} dokumentiert) und die Säurezahl weniger als 0,5 mg KOH/g betrug, wurde der Ansatz abgekühlt. Der auf Raumtemperatur abgekühlte Ansatz wurde in einen Rührkolben mit Rührer, Thermometer, Tauchrohr, Claisen-Brücke und Vorlagekolben umgefüllt. Die Apparatur wurde mindestens eine Stunde mit Stickstoff (6 l/h) über das Tauchrohr gespült. Anschließend wurde der restliche Überschussalkohol bei T³ durch Anlegen von Vakuum abdestilliert. Sobald der Restgehalt an Alkohol R¹OH gemäß GC-Chromatogramm unterhalb von 5 Mol-% - bezogen auf die Überschussmenge s₁ des Alkohols R¹OH - betrug, wurde die Heizung abgestellt und die Reaktion unter Vakuum und Einleiten von Stickstoff (20mbar) auf 80°C abgekühlt. Vom Kolbeninhalt wurde die Säurezahl bestimmt. Entsprechend des Ergebnisses wurde das Reaktionsmedium durch langsames Zutropfen der dreifachen stöchiometrischen Menge an Base (10 %-ige wässrige NaOH-Lösung) neutralisiert und unter Einleitung von Stickstoff (6 l/h) bei 80 °C für 15 min gerührt. Anschließend wurde der Ansatz langsam vom Umgebungsdruck bis auf ca. 1 mbar evakuiert, woraufhin auf ca. 120 °C aufgeheizt wurde und bei konstanter Temperatur restliche flüchtige Bestandteile mithilfe einer Stickstoffeinleitung abgetrennt wurden. Der Stickstoffstrom wurde so eingestellt, dass der Druck nicht über 20 mbar stieg. Sobald der Restalkoholgehalt entsprechend GC-Analyse kleiner als 0,025 Flächen-% war, wurde die Heizung abgestellt und unter Vakuum und Einleiten von Stickstoff auf 80 °C abgekühlt. Bei dieser Temperatur wurde das Produkt über einen Büchnertrichter mit Filterpapier und vorgepresstem Filterkuchen aus Filterhilfsmittel (Perlite Typ D14) mittels Vakuum in eine Saugflasche filtriert. An dem Filtrat wurde eine GC-Analyse durchgeführt, anhand derer die Reinheit (R) und die Zusammensetzung des Produkts analysiert wurde.

**Tabelle 9: gemessene und berechnete Werte zum Beispiel 9**

| | |
|---|---|
| R¹OH = *Iso*pentanol | m₁ + s₁ = 661 g |
| Stoffmenge R¹OH = Stoffmenge R¹-Äquivalente (C₅) | 7,5 mol |
| R²OH = *Iso*nonanol | m₂ = 720 g |
| Stoffmenge R²OH = Stoffmenge R²-Äquivalente (C₉) | 5 mol |
| B = Bernsteinsäure | m_{b} = 591 g |
| (Sigma Aldrich, Reinheit >99 %) | |
| Stoffmenge B | 5 mol |
| → Stoffmenge Esterfunktionen | 10 mol |
| Erwarteter Molanteil der R¹-Funktionen an R¹ und R²-Funktionen im Estergemisch (bei vollständigem Einbau von R²) | 10 mol - 5 mol = 5 mol |
| | entsprechend 50 Mol-% |
| Verhältnis m₁ : m₂ | 0,5 : 0,5 |
| s₁ | 7,5 mol - (10 mol - 5 mol) = 2,5 mol |
| T¹ → T² (Sumpftemperatur Anfang → Ende) | 120 °C → 218 °C |
| m_{d1} (Destillat Reaktion) | 184 g (10,2 Mol Wasser) |
| m_{sch} | 0 g |
| T³ | 177 °C |
| R (Reinheit) | > 99,9 % |
| Statistische Erwartungswerte für vollständigen Einbau von C₉: | **25,0** : **50,0** : **25,0** |
| (C₅/C₅) : (C₅/C₉) : (C₉/C₉) | (erwarteter C₅-Anteil: 50 %) |
| Zusammensetzung gemäß GC | **26,9** : **50,9** : **22,2** (C₅-Anteil: 52,3 %) |
| (C₅/C₅) : (C₅/C₉) : (C₉/C₉) | Abweichung: |
| | \|26,9-25,0\| + \|50,9-50,0\| + \|22,2-25,0\| = 5,6 |

Es wurden 1200 g aufgearbeitetes Estergemisch erhalten.

### Beispiel 10 (nicht erfindungsgemäß):

Veresterung von 1,2-Cyclohexandicarbonsäureanhydrid mit Isopentanol und Isononanol (27 : 49 : 24)

Das Beispiel 10 wurde ausgeführt wie für das Beispiel 9 beschrieben.

**Tabelle 10: gemessene und berechnete Werte zum Beispiel 10**

| | |
|---|---|
| R¹OH = *Iso*pentanol | m₁ + s₁ = 264 g |
| Stoffmenge R¹OH = Stoffmenge R¹-Äquivalente (C₅) | 3 mol |
| R²OH = *Iso*nonanol | m₂ = 288 g |
| Stoffmenge R²OH = Stoffmenge R²-Äquivalente (C₉) | 2 mol |
| B = 1,2- Cyclohexandicarbonsäureanhydrid | m_{b} = 308 g |
| (Sigma Aldrich, Reinheit 95 %) | |
| Stoffmenge B | 2 mol |
| → Stoffmenge Esterfunktionen | 4 mol |
| Erwarteter Molanteil der R¹-Funktionen an R¹ und R²-Funktionen im Estergemisch (bei vollständigem Einbau von R²) | 4 mol - 2 mol = 2 mol |
| | entsprechend 50 Mol-% |
| Verhältnis m₁ : m₂ | 0,5 : 0,5 |
| s₁ | 3 mol - (4 mol - 2 mol) = 1 mol |
| T¹ → T² (Sumpftemperatur Anfang → Ende) | 164 °C → 217 °C |
| m_{d1} (Destillat Reaktion) | 36 g (2 mol Wasser) |
| m_{sch} | 0 g |
| T³ | 160 °C |
| R (Reinheit) | > 99,9 % |
| Statistische Erwartungswerte für vollständigen Einbau von C₉: | **25,0** : **50,0** : **25,0** |
| (C₅/C₅) : (C₅/C₉) : (C₉/C₉) | (erwarteter C₅-Anteil: 50 %) |
| Zusammensetzung gemäß GC | **26,8: 48,7 : 24,4** (C₅-Anteil: 51,2 %) |
| (C₅/C₅) : (C₅/C₉) : (C₉/C₉) | Abweichung: |
| | \|26,8-25,0\| + \|48,7-50,0\| + \|24,4-25,0\| = 3,7 |

Es wurden 540 g aufgearbeitetes Estergemisch erhalten.

### Beispiel 11 (nicht erfindungsgemäß):

Veresterung von Phthalsäureanhydrid mit Isononanol und Isotridecanol

Das Beispiel 11 wurde ausgeführt wie für das Beispiel 9 beschrieben.

**Tabelle 11: gemessene und berechnete Werte zum Beispiel 11**

| | |
|---|---|
| R²OH = Isononanol | m₁ + s₁ = 864 g |
| Stoffmenge R²OH = Stoffmenge R²-Äquivalente (C₉) | 6 mol |
| R²OH = Isotridecanol | m₂ = 801 g |
| (Evonik Industries AG, Reinheit >99 %) | |
| Stoffmenge R²OH = Stoffmenge R²-Äquivalente (C₁₃) | 4 mol |
| B = Phthalsäureanhydrid | m_{b} = 592 g |
| (Sigma Aldrich, Reinheit 99 %) | |
| Stoffmenge B | 4 mol |
| → Stoffmenge Esterfunktionen | 8 mol |
| Erwarteter Molanteil der R¹-Funktionen an R¹ und R²-Funktionen im Estergemisch (bei vollständigem Einbau von R²) | 8 mol - 4 mol = 4 mol |
| | entsprechend 50 Mol-% |
| Verhältnis m₁ : m₂ | 0,5 : 0,5 |
| s₁ | 6 mol - (8 mol - 4 mol) = 2 mol |
| T¹ → T² (Sumpftemperatur Anfang → Ende) | 212 °C → 240 °C |
| m_{d1} (Destillat Reaktion) | 73 g (4 mol Wasser) |
| m_{Sch} | 70 g |
| T³ | 200 °C |
| R (Reinheit) | > 99,9 % |
| Statistische Erwartungswerte für vollständigen Einbau von C₉: | **25,0** : **50,0** : **25,0** |
| (C₉/C₉) : (C₉/C₁₃) : (C₁₃/C₁₃) | (erwarteter C₉-Anteil: 50 %) |
| Zusammensetzung gemäß GC | nicht bestimmbar aufgrund von Signalüberlagerung im GC |
| (C₉/C₉) : (C₉/C₁₃) : (C₁₃/C₁₃) | |

Es wurden 1580 g aufgearbeitetes Estergemisch erhalten.

### Beispiel 12 (nicht erfindungsgemäß):

Veresterung von Zitronensäure mit Isopentanol und Isononanol (54 : 39 : 7 : 0)

Das Beispiel 12 wird ausgeführt wie für das Beispiel 9 beschrieben, wobei jedoch als Katalysator anstelle des Tetra-n-Butyltitanates 0,30 Gew.-% Methansulfonsäure (Sigma Aldrich, Reinheit ≥ 99,5 %) (bezogen auf die Masse der Säure) hinzugegeben wurde. Die Reaktion wurde in diesem Beispiel durch Abkühlen abgebrochen, sobald die Säurezahl einen Wert von 1,0 mg KOH pro g des Rohproduktes unterschritten hatte und die erwartete Menge an Reaktionswasser erhalten wurde. Vor dem Abfiltrieren wurde das Rohprodukt für 60 Minuten bei 80 °C mit 2 Gew.-% basischem Aluminiumoxid (Gew.-% bezogen auf die Gesamtmasse) gerührt.

**Tabelle 12: gemessene und berechnete Werte zum Beispiel 12**

| | |
|---|---|
| R¹OH = Isopentanol | m₁ + s₁ = 1057 g |
| Stoffmenge R¹OH = Stoffmenge R¹-Äquivalente (C₅) | 12 mol |
| R²OH = Isononanol | m₂ = 346 g |
| Stoffmenge R²OH = Stoffmenge R²-Äquivalente (C₉) | 2,4 mol |
| B = Zitronensäure Monohydrat | m_{b} = 841 g |
| (Sigma Aldrich, Reinheit 99%) | |
| Stoffmenge B | 4 mol |
| → Stoffmenge Esterfunktionen | 12 mol |
| Erwarteter Molanteil der R¹-Funktionen an R¹ und R²-Funktionen im Estergemisch (bei vollständigem Einbau von R²) | 12 mol - 2,4 mol = 9,6 mol |
| | entsprechend 80 Mol-% |
| Verhältnis m₁ : m₂ | 0,8 : 0,2 |
| s₁ | 12 mol - (12 mol - 2,4 mol) = 2,4 mol |
| T¹ → T² (Sumpftemperatur Anfang -> Ende) | 125 °C → 161 °C |
| m_{d1} (Destillat Reaktion) | 295 g (16,4 mol Wasser) |
| m_{Sch} | 150 g |
| T³ | 160 °C |
| R (Reinheit) | 99,5 % |
| Statistische Erwartungswerte für vollständigen Einbau von Cg: | **51,2** : **38,4** : **9,6** : **0,8** |
| (C₅/C₅) : (C₅/C₉) : (C₉/C₉) | (erwarteter Cs-Anteil: 80 %) |
| Zusammensetzung gemäß GC | **53,9** : **39,4** : **6,7** : **<0,1** |
| (C₅/C₅) : (C₅/C₉) : (C₉/C₉) | (Cs-Anteil: 82,4 %) |
| | Abweichung: |
| | \|53,9-51,2\| + \|39,4-38,4\| + \|6,7-9,6\| + 0,8 = 7,4 |

Es wurden 1080 g aufgearbeitetes Estergemisch erhalten.

### Beispiel 13:

Herstellung von Plastisolen der erfindungsgemäßen Estergemische 3:27:70 (**1**, Beispiel 8), 13:46:41 (**2**, Beispiel 3), 24:51:25 (**3**, Beispiel 4) und 44:45:11 (**4**, Beispiel 7)

Es wurden PVC-Plastisole hergestellt, wie sie beispielsweise zur Fertigung von Deckstrichfilmen für Fußbodenbeläge verwendet werden. Die Angaben in den Plastisolrezepturen sind jeweils in Massenanteilen. Die Rezepturen der Polymerzusammensetzungen sind in Tabelle 13 aufgelistet.

**Tabelle 13: Plastisolrezeptur**

| | **P1** | **P2** | **P3** | **P4** |
|---|---|---|---|---|
| PVC (Vestolit B 7021 - Ultra; Fa. Vestolit) | 100 | 100 | 100 | 100 |
| Estergemisch 3:27:70 (**1**) | 50 | | | |
| Estergemisch 13:46:41 (**2**) | | 50 | | |
| Estergemisch 24:51:25 (**3**) | | | 50 | |
| Estergemisch 44:45:11 (**4**) | | | | 50 |
| epoxidiertes Sojabohnenöl als Co-Stabilisator (Drapex 39, Fa. Galata) | 3 | 3 | 3 | 3 |
| Thermostabilisator auf Ca/Zn-Basis (Mark CZ 149, Fa. Galata) | 2 | 2 | 2 | 2 |

| | | | | |
|---|---|---|---|---|
| Angaben in phr (phr = parts per hundred parts resin); P: Plastisol | | | | |

Die Estergemische wurden vor der Zugabe auf 25 °C temperiert. Zuerst wurden die flüssigen und dann die pulverförmigen Bestandteile in einen PE-Becher eingewogen. Von Hand wurde die Mischung mit einem Salbenspatel so eingerührt, dass kein unbenetztes Pulver mehr vorhanden war. Der Mischbecher wurde dann in die Klemmvorrichtung eines Dissolverrührers eingespannt. Vor dem Eintauchen des Rührers in die Mischung wurde die Drehzahl auf 1800 Umdrehungen pro Minute eingestellt. Nach dem Einschalten des Rührers wurde so lange gerührt, bis die Temperatur an der Digitalanzeige des Thermofühlers 30,0 °C erreichte. Damit war sichergestellt, dass die Homogenisierung des Plastisols bei einem definierten Energieeintrag erreicht wurde. Danach wurde das Plastisol sofort für weitere Untersuchungen in einem Klimaschrank auf 25,0 °C temperiert.

### Beispiel 14:

### Geliertemperatur der Plastisole 1 bis 4 (P1 - P4)

Die Untersuchung des Gelierverhaltens der Plastisole wurde mit einem Physica MCR 101 im Oszillationsmodus mit einem Platte-Platte Messsystem (PP25), welches schubspannungsgesteuert betrieben wurde, vorgenommen. Eine zusätzliche Temperierhaube wurde an das Gerät angeschlossen, um eine homogene Wärmeverteilung und eine gleichmäßige Probentemperatur zu erreichen.

Folgende Parameter wurden eingestellt:

| Modus: | Temperatur-Gradient |
|---|---|
| Start-Temperatur: | 25 °C |
| End-Temperatur: | 180 °C |
| Heiz/Kühlrate: | 5 °C / min |
| Oszillations-Frequenz: | 4 - 0,1 Hz Rampe logarithmisch |
| Kreisfrequenz Omega: | 10 1 / s |
| Anzahl Messpunkte: | 63 |
| Messpunktdauer: | 0,5 min |
| Automatische Spaltnachführung | F : 0 N |
| Konstante Messpunktdauer Spaltweite | 0,5 mm |

Durchführung der Messung:
Auf die untere Messsystemplatte wurden mit dem Spatel einige Gramm des zu messenden Plastisols luftblasenfrei aufgetragen. Dabei wurde darauf geachtet, dass nach dem Zusammenfahren des Messsystems etwas Plastisol gleichmäßig aus dem Messsystem herausquellen konnte (nicht mehr als 6 mm rundum). Anschließend wurde die Temperierhaube über der Probe positioniert und die Messung gestartet. Bestimmt wurde die sogenannte komplexe Viskosität des Plastisols nach 24 h (Lagerung des Plastisols bei 25 °C in einem Temperierschrank der Fa. Memmert) in Abhängigkeit von der Temperatur.

Als Maß für die Gelierung wurde ein deutlicher Anstieg der komplexen Viskosität betrachtet. Als Vergleichswert wurde daher die Temperatur bei Erreichen einer Plastisolviskosität von 1000 Pa s verwendet.

**Tabelle 14: Gelierung der Plastisole 1 bis 4 nach 24 h, Temperatur in °C bei Erreichen einer Plastisolviskosität von 10³ Pa s (kurz: Geliertemperatur)**

| | Geliertemperatur [°C] |
|---|---|
| Diisononylterephthalat (DINT) | 109 |
| **P1** (3:27:70) | 92 |
| **P2** (13:46:41) | 83 |
| **P3** (24:51:25) | 80 |
| **P4** (44:45:11) | 76 |
| Diisopentylterephthalat (DPT) | 70 |

| | |
|---|---|
| P: Plastisol | |

In der Abbildung 1 ist für die erfindungsgemäßen Estergemische **1** bis **4** die Geliertemperatur des Plastisols aus der Tabelle 14 gegen die zugehörige Flüchtigkeit der dasselbe Estergemisch enthaltenen Folie (aus Beispiel 16, Tabelle 15) aufgetragen.

### Beispiel 15:

### Herstellung von Folien der erfindungsgemäßen Estergemische

Die im Beispiel 13 hergestellten Plastisole wurden jeweils zu 1 mm dicken Folien verarbeitet.

Dazu wurde zunächst Hochglanztrennpapier (Fa. Sappi, Italien) auf eine Größe von 30 x 44 cm zugeschnitten und im Spannrahmen der Streicheinrichtung LTSV für den Mathis-Ofen eingelegt. Danach wurde der Spannrahmen auf den Führungsrahmen aufgelegt, der Mathis-Ofen (Typ LTF) auf 200 °C eingestellt und der Rahmen nach Erreichen dieser Temperatur 15 Sekunden vorgewärmt. Danach wurde die Rakel in die Einspannvorrichtung gelegt und der Rakelspalt über Vorversuche so eingestellt, dass die Foliendicke nach Abschluss der Gelierung 1 mm (+/- 0,05 mm) betrug. Auf den vorderen Rand des Papiers wurde ein Klebestreifen angebracht, um überschüssige Paste aufzufangen. Danach wurde die Paste vor der Rakel aufgetragen und diese durch Ziehen des Führungsrahmens mit der Rakel über das eingespannte Trennpapier verstrichen (ca. 3 m/min Geschwindigkeit). Danach wurde die Rakel herausgenommen und der Klebestreifen mit der überschüssigen Paste abgenommen. Anschließend wurde die Schmelzwalze abgesenkt und der Spannrahmen in den Ofen eingefahren. Nach der Gelierung (2 Minuten bei 200 °C) wurde der Rahmen wieder aus dem Ofen herausgefahren und nach Abkühlen die Folie von dem Papier abgezogen.

Aus den Folien wurden 3 Kreise von 10 cm² pro zu prüfender Rezeptur ausgestanzt. Zusätzlich wurden die Kreise mit der Schere radial angeschnitten (2 Schnitte a 5 mm). Die Kreise wurden für eine halbe Stunde im Exsikkator (gefüllt mit KC- Trockenperlen orange) klimatisiert und anschließend ausgewogen.

### Beispiel 16:

### Massenverlust bei Aktivkohlelagerung bei den Folien 1 - 4 (F1 - F4)

Weißblechdosen (1 L, hohe Form) wurden, damit ein Druckaustausch stattfinden konnte, im Deckel mit einem Loch versehen. Der Boden der Weißblechdosen wurde mit 120 mL Aktivkohle bedeckt. Die in diesem Test eingesetzte Aktivkohle (Nr. 774408 der Fa. Roth) wurde vorher in einer Abdampfschale für 6 Stunden im Trockenschrank bei 100 +/-1 °C getrocknet und nach kurzer Abkühlung eingesetzt. Auf die Aktivkohle wurde der erste Probenkreis mittig platziert. Weitere 120 mL Aktivkohle wurden auf den Probenkreis gegeben. Insgesamt wurden die Weißblechdosen mit 480 mL Aktivkohle und 3 Probenkreisen schichtweise befüllt. Der Deckel der Weißblechdosen wurde ohne Druck auf die Dosen aufgelegt.

Die befüllten Weißblechdosen wurden bei 100 +/-1 °C für 3 Tage im Temperierschrank gelagert. Nach der Lagerung wurde die Aktivkohle von den Kreisen mittels Analysenpinsel entfernt, die Kreise zum Abkühlen 30 Minuten in einem Exsikkator gelagert und anschließend ausgewogen.

Nach der Auswaage wurden die Probenkreise wieder mit Aktivkohle zurück in die Weißblechdosen geschichtet. Hierzu wurde darauf geachtet, dass die Probenkreise wieder derselben Aktivkohle und derselben Dose zugeordnet wurden. Die Dosen wurden erneut im Temperierschrank platziert. Nach insgesamt 7 Tagen wurden die Proben dann, wie bereits beschrieben, nochmals ausgewogen.

Es wurde die prozentuale Massenveränderung von jedem Probenkreis berechnet und der Mittelwert über die 3 Kreise je Rezeptur berechnet.

**Tabelle 15: Massenverlust bei Aktivkohlelagerung in Massen-% (Flüchtigkeit)**

| | Flüchtigkeit [Massen-%] | |
|---|---|---|
| | 3 Tage | 7 Tage |
| Diisononylterephthalat (DINT) | 4,0 | 5,4 |
| **F1** (3:27:70) | 4,9 | 8,2 |
| **F2** (13:46:41) | 6,2 | 11,0 |
| **F3** (24:51:25) | 9,3 | 15,3 |
| **F4** (44:45:11) | 13,9 | 20,8 |
| Diisopentylterephthalat (DPT) | 20,5 | 26,2 |

| | | |
|---|---|---|
| F: Folie | | |

In der Abbildung 1 ist für die erfindungsgemäße Estergemische **1** bis **4** die Geliertemperatur des Plastisols aus der Tabelle 14 des Beispiels 14 gegen die zugehörige Flüchtigkeit der dasselbe Estergemisch enthaltenen Folie (aus Tabelle 15) aufgetragen.

## Patentansprüche

1. Verfahren zur Herstellung von Estergemischen umfassend A(COOR¹)(COOR¹)ₓ, A(COOR¹)(COOR²)ₓ, A(COOR²)(COOR¹)ₓ und A(COOR²)(COOR²)ₓ durch Umsetzung des Esters A(COOR)ₓ₊₁ mit der Einschränkung, dass der Alkohol ROH des Restes R einen niedrigeren Siedepunkt bei einem bestimmten Druck aufweist als der Alkohol R¹OH des Restes R¹ bei demselben Druck, mit einer Menge (m₁ + s₁) R¹OH und einer Menge m₂ R²OH, wobei das Reaktionsgemisch zum Sieden erhitzt wird und
A für einen aromatischen, alicyclischen oder aliphatischen Rest steht,
x 1 ist,
R¹ und R² unabhängig voneinander für substituierte oder nichtsubstituierte Arylreste oder lineare oder verzweigte, substituierte oder nicht substituierte Alkylreste mit 3 bis 20 Kohlenstoffatomen stehen, wobei der Alkohol R¹OH einen niedrigeren Siedepunkt bei einem bestimmten Druck aufweist als der Alkohol R²OH bei demselben Druck,
m₁ und m₂ den Mol-Äquivalenten der in der eingesetzten Säure, dem eingesetzten Anhydrid oder dem eingesetzten Ester einzuführenden Alkoholresten OR¹ und OR² entspricht, und
s₁ kleiner als m₁ + m₂ und gleichzeitig größer als 0,05 · (m₁ + m₂) ist,
**dadurch gekennzeichnet, dass** in dem Verfahren ein Katalysator eingesetzt wird und im Reaktionsgemisch der Gehalt an R¹OH auf weniger als 10 Mol-% - bezogen auf die Überschussmenge s₁ des Alkoholes R¹OH - abgesenkt wurde, bevor der Katalysator zerstört wird.

2. Verfahren gemäß Anspruch 1, **dadurch gekennzeichnet, dass** es sich um ein Estergemisch der Phthalsäure, der Terephthalsäure, der Isophthalsäure, der 1,2-, 1,3- oder 1,4- Cyclohexandicarbonsäure, der Adipinsäure, der Sebacinsäure, der Maleinsäure, der Bernsteinsäure und der Furandicarbonsäure handelt.

3. Verfahren gemäß einem der Ansprüche 1 oder 2, **dadurch gekennzeichnet, dass** R¹ und R² unabhängig voneinander ausgewählt sind aus Alkylresten, welche 3 bis 20 Kohlenstoffatome enthalten.

4. Verfahren gemäß einem der Ansprüche 1 bis 3, **dadurch gekennzeichnet, dass** R¹ und R² unabhängig voneinander ausgewählt sind aus Propyl-, Butyl-, tert-Butyl-, Isobutyl-, 2-Methylbutyl-, 3-Methylbutyl-, n-Pentyl-, Isopentyl-, Hexyl-, Heptyl-, Isoheptyl-, Octyl-, Isooctyl-, 2-Ethylhexyl-, Nonyl-, n-Nonyl-, Isononyl-, Decyl-, Isodecyl-, 2-Propyl-heptyl-, Undecyl- und Tridecyl- Resten.

5. Verfahren gemäß einem der Ansprüche 1 bis 4, **dadurch gekennzeichnet, dass** die Siedepunkte der Alkohole R¹OH und R²OH sich um mindestens 10 °C unterscheiden.

6. Verfahren gemäß einem der Ansprüche 1 bis 5, **dadurch gekennzeichnet, dass** es ein Estergemisch bereitstellt, in welchem das Molverhältnis der Ester A(COOR¹)(COOR¹)ₓ, A(COOR¹)(COOR²)ₓ, A(COOR²)(COOR¹)ₓ und A(COOR²)(COOR²)ₓ für x = 1 um weniger als 15 Punkte vom statistisch ermittelten Erwartungswert abweicht, welcher sich ergibt, wenn man von einem vollständigen Einbau der Alkoholreste OR² ausgeht, wobei dieser Punkte-Wert der Summe aller Beträge der Differenzen zwischen statistischem Erwartungswert und tatsächlichem Mol-Anteil jedes einzelnen Esters im Estergemisch entspricht für den Fall, dass sich die Summe der Mol-Anteile der oben genannten Ester im Estergemisch zu 100 addiert.

7. Verfahren gemäß einem der Ansprüche 1 bis 6, **dadurch gekennzeichnet, dass** es sich um ein Estergemisch der Phthalsäure, der Terephthalsäure oder der Isophthalsäure handelt und das Estergemisch nach einer optionalen Aufarbeitung in einem nachfolgenden Verfahrensschritt hydriert wird.

8. Verfahren nach Anspruch 1, **dadurch gekennzeichnet, dass** das Verhältnis m₁ zu m₂ (m₁ : m₂) im Bereich von 1 : 9 bis 9 : 1 liegt.

9. Verwendung eines Verfahrens gemäß einem der Ansprüche 1 bis 7 zur Einstellung verarbeitungs- und/oder anwendungsrelevanter Eigenschaften eines Estergemisches durch Steuerung der Mengenverteilung der Ester im Estergemisch.

## Claims

1. Process for producing ester mixtures comprising A(COOR¹)(COOR¹)ₓ, A(COOR¹)(COOR²)ₓ, A(COOR²)(COOR¹)ₓ and A(COOR²)(COOR²)ₓ by reaction of the ester A(COOR)ₓ₊₁ with the restriction that the alcohol ROH of the radical R has a lower boiling point at a defined pressure than the alcohol R²OH of the radical R¹ at the same pressure, with an amount (m₁ + s₁ of R²OH and an amount m₂ of R²OH, wherein the reaction mixture is heated to boiling, and
A is an aromatic, alicyclic or aliphatic radical,
x is 1,
R¹ and R² independently of one another are substituted or unsubstituted aryl radicals or linear or branched, substituted or unsubstituted alkyl radicals having 3 to 20 carbon atoms, the alcohol R²OH having a lower boiling point at a defined pressure than the alcohol R²OH at the same pressure,
m₁ and m₂ correspond to the mole equivalents of the alcohol radicals OR¹ and OR² to be introduced in the acid used, the anhydride used or the ester used, and
s₁ is less than m₁ + m₂ and at the same time is greater than 0.05·(m₁ + m₂),
**characterized in that** a catalyst is used in the process and the amount of R²OH in the reaction mixture has been lowered to less than 10 mol%, based on the excess amount s₁ of the alcohol R²OH, before the catalyst is destroyed.

2. Process according to Claim 1, **characterized in that** the ester mixture is a mixture of phthalic acid, terephthalic acid, isophthalic acid, 1,2-, 1,3- or 1,4-cyclohexanedicarboxylic acid, adipic acid, sebacic acid, maleic acid, succinic acid and furandicarboxylic acid.

3. Process according to either of Claims 1 and 2, **characterized in that** R¹ and R² independently of one another are selected from alkyl radicals which contain 3 to 20 carbon atoms.

4. Process according to any of Claims 1 to 3, **characterized in that** R¹ and R² independently of one another are selected from propyl, butyl, tert-butyl, isobutyl, 2-methylbutyl, 3-methylbutyl, n-pentyl, isopentyl, hexyl, heptyl, isoheptyl, octyl, isooctyl, 2-ethylhexyl, nonyl, n-nonyl, isononyl, decyl, isodecyl, 2-propylheptyl, undecyl and tridecyl radicals.

5. Process according to any of Claims 1 to 4, **characterized in that** the boiling points of the alcohols R²OH and R²OH differ by at least 10°C.

6. Process according to any of Claims 1 to 5, **characterized in that** it provides an ester mixture in which the molar ratio of the esters A(COOR¹)(COOR¹)ₓ, A(COOR¹)(COOR²)ₓ, A(COOR²)(COOR¹)ₓ and A(COOR²)(COOR²)ₓ for x = 1 deviates by less than 15 points from the statistically determined expectation value which arises on assumption of complete incorporation of the alcohol radicals OR², this points value corresponding to the sum total of all amounts of the differences between statistical expectation value and actual molar fraction of each individual ester in the ester mixture in the event that the sum total of the molar fractions of the abovementioned esters in the ester mixture adds up to 100.

7. Process according to any of Claims 1 to 6, **characterized in that** the ester mixture is a mixture of phthalic acid, terephthalic acid or isophthalic acid and after optional work-up the ester mixture is hydrogenated in a subsequent process step.

8. Process according to Claim 1, **characterized in that** the ratio of m₁ to m₂ (m₁:m₂) is in the range from 1:9 to 9:1.

9. Use of a process according to any of Claims 1 to 7 for setting processing-relevant and/or application-relevant properties of an ester mixture by controlling the quantitative distribution of the esters in the ester mixture.

## Revendications

1. Procédé pour la préparation de mélanges d'esters comprenant A(COOR¹)(COOR¹)ₓ, A(COOR¹)(COOR²)ₓ, A(COOR²)(COOR¹)ₓ et A(COOR²)(COOR²)ₓ par transformation de l'ester A(COOR)ₓ₊₁ avec la restriction que l'alcool ROH du radical R présente un point d'ébullition plus bas, à une pression déterminée, que l'alcool R¹OH du radical R¹ à la même pression, avec une quantité (m₁ + s₁) de R²OH et une quantité m₂ de R²OH, le mélange réactionnel étant chauffé à ébullition et où
A représente un radical aromatique, alicyclique ou aliphatique,
x vaut 1,
R¹ et R² représentent, indépendamment l'un de l'autre, des radicaux aryle substitués ou non substitués ou des radicaux alkyle linéaires ou ramifiés, substitués ou non substitués, comprenant 3 à 20 atomes de carbone, l'alcool R²OH présentant un point d'ébullition plus bas, à une pression déterminée, que l'alcool R²OH à la même pression,
m₁ et m₂ correspondent aux équivalents en mole des radicaux alcool OR¹ et OR² à introduire dans l'acide utilisé, dans l'anhydride utilisé ou dans l'ester utilisé et
s₁ est inférieur à m₁ + m₂ et simultanément supérieur à 0,05 * (m₁ + m₂),
caractérisé en qu'un catalyseur est utilisé dans le procédé et, dans le mélange réactionnel, la teneur en R¹OH a été abaissée à moins de 10% en mole - par rapport à la quantité en excès s₁ de l'alcool R¹OH - avant la dégradation du catalyseur.

2. Procédé selon la revendication 1, **caractérisé en ce qu'**il s'agit d'un mélange d'esters de l'acide phtalique, de l'acide téréphtalique, de l'acide isophtalique, de l'acide 1,2-cyclohexanedicarboxylique, 1,3-cyclohexanedicarboxylique ou 1,4-cyclohexanedicarboxylique, de l'acide adipique, de l'acide sébacique, de l'acide maléique, de l'acide succinique et de l'acide furannedicarboxylique.

3. Procédé selon l'une quelconque des revendications 1 ou 2, **caractérisé en ce que** R¹ et R² sont choisis, indépendamment l'un de l'autre, parmi les radicaux alkyle qui contiennent 3 à 20 atomes de carbone.

4. Procédé selon l'une quelconque des revendications 1 à 3, **caractérisé en ce que** R¹ et R² sont choisis, indépendamment l'un de l'autre, parmi les radicaux propyle, butyle, tert-butyle, isobutyle, 2-méthylbutyle, 3-méthylbutyle, n-pentyle, isopentyle, hexyle, heptyle, isoheptyle, octyle, isooctyle, 2-éthylhexyle, nonyle, n-nonyle, isononyle, décyle, isodécyle, 2-propylheptyle, undécyle et tridécyle.

5. Procédé selon l'une quelconque des revendications 1 à 4, **caractérisé en ce que** les points d'ébullition des alcools R²OH et R²OH diffèrent d'au moins 10°C.

6. Procédé selon l'une quelconque des revendications 1 à 5, **caractérisé en ce qu'**il permet de préparer un mélange d'esters, dans lequel le rapport molaire des esters A(COOR¹)(COOR¹)ₓ, A(COOR¹)(COOR²)ₓ, A(COOR²)(COOR¹)ₓ et A(COOR²)(COOR²)ₓ pour x = 1 s'écarte de moins de 15 points de la valeur attendue, déterminée statistiquement, qui est obtenue lorsqu'on suppose une incorporation complète des radicaux alcool OR², cette valeur de points correspondant à la somme de toutes les contributions des différences entre la valeur attendue statistique et la proportion molaire effective de chaque ester individuel dans le mélange d'esters pour le cas où la somme des proportions molaires des esters susmentionnés dans le mélange d'esters vaut 100.

7. Procédé selon l'une quelconque des revendications 1 à 6, **caractérisé en ce qu'**il s'agit d'un mélange d'esters de l'acide phtalique, de l'acide téréphtalique ou de l'acide isophtalique et le mélange d'esters est hydrogéné après un traitement facultatif dans une étape de procédé consécutive.

8. Procédé selon la revendication 1, **caractérisé en ce que** le rapport m₁ à m₂ (m₁:m₂) se situe dans la plage de 1:9 à 9:1.

9. Utilisation d'un procédé selon l'une quelconque des revendications 1 à 7 pour le réglage de propriétés pertinentes pour la mise en œuvre et/ou l'utilisation d'un mélange d'esters par régulation de la répartition des quantités des esters dans le mélange d'esters.
